(19) Europäisches Patentamt
European Patent Office
Office européen des brevets

(11) **EP 3 903 863 B1**

(12) **EUROPÄISCHE PATENTSCHRIFT**

(45) Veröffentlichungstag und Bekanntmachung des Hinweises auf die Patenterteilung:
**06.04.2022   Patentblatt 2022/14**

(21) Anmeldenummer: **21169787.5**

(22) Anmeldetag: **22.04.2021**

(51) Internationale Patentklassifikation (IPC):
**A61M 16/00** (2006.01)   **A61M 16/08** (2006.01)
**A61M 16/10** (2006.01)   **G01M 3/22** (2006.01)
**G01N 33/00** (2006.01)   *A61M 16/18* (2006.01)
*A61M 16/20* (2006.01)   *A61M 16/22* (2006.01)
**G01M 3/00** (2006.01)

(52) Gemeinsame Patentklassifikation (CPC):
(C-Sets verfügbar)
**A61M 16/024; A61B 5/083; A61M 16/085;
A61M 16/0891; A61M 16/104; G01M 3/228;
G01N 33/004;** A61M 16/0051; A61M 16/0057;
A61M 16/0066; A61M 16/0078; A61M 16/0808;
A61M 16/0833; A61M 16/1055; A61M 16/106;
(Forts.)

(54) **VERFAHREN UND VORRICHTUNG ZUR DETEKTION EINES LECKS IN EINEM BEATMUNGSKREISLAUF**

METHOD AND DEVICE FOR DETECTING A LEAK IN A VENTILATION CIRCUIT

PROCÉDÉ ET DISPOSITIF DE DÉTECTION D'UNE FUITE DANS UN CIRCUIT RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.04.2020   DE 102020002570**

(43) Veröffentlichungstag der Anmeldung:
**03.11.2021   Patentblatt 2021/44**

(73) Patentinhaber: **Drägerwerk AG & Co. KGaA
23558 Lübeck (DE)**

(72) Erfinder:
• **Peter, Gerd
23558 Lübeck (DE)**
• **Kroh, Martin
23558 Lübeck (DE)**

(56) Entgegenhaltungen:
**EP-A1- 1 961 439       EP-A1- 1 974 763
WO-A1-2014/068000       WO-A1-2018/112588**

DE-A1-102017 011 625     US-A1- 2019 099 542
US-B2- 8 424 529     US-B2- 8 752 544

• VISHNOI R ET AL: "ADAPTIVE CONTROL OF CLOSED-CIRCUIT ANESTHESIA", IEEE TRANSACTIONS ON BIOMEDICAL ENGINEERING, IEEE SERVICE CENTER, PISCATAWAY, NJ, USA, Bd. 38, Nr. 1, 1. Januar 1991 (1991-01-01) , Seiten 39-46, XP000225246, ISSN: 0018-9294, DOI: 10.1109/10.68207

(52) Gemeinsame Patentklassifikation (CPC): (Forts.)
A61M 16/18; A61M 16/205; A61M 16/208;
A61M 16/209; A61M 16/22; A61M 2016/0024;
A61M 2016/0027; A61M 2016/003;
A61M 2016/1025; A61M 2016/103;
A61M 2016/1035; A61M 2202/0208;
A61M 2202/0225; A61M 2202/0283; A61M 2205/15;
A61M 2205/18; A61M 2205/3331; A61M 2205/3358;
A61M 2205/50; A61M 2205/7518; A61M 2205/7527;
A61M 2230/432; A61M 2230/435; A61M 2230/437

C-Sets

A61M 2230/432;
A61M 2230/435;
A61M 2230/437

## Beschreibung

Die Erfindung betrifft ein Verfahren und eine Vorrichtung, um während einer künstlichen Beatmung eines Patienten automatisch eine Leckage zu erkennen, wobei diese Leckage die Messung von Gaskonzentrationen, die in einer Fluidverbindung zwischen einem medizinischen Gerät und dem Patienten auftreten, beeinflussen kann.

Um einen Patienten künstlich zu beatmen und optional zu sedieren, wird eine Fluidverbindung zwischen einem Beatmungsgerät und dem Patienten hergestellt. Bei der künstlichen Beatmung führt das Beatmungsgerät dem Patienten über die Fluidverbindung ein Atemgas zu, und das ausgeatmete Atemgas wird vom Patienten weggeführt. Das Beatmungsgerät kann als ein Anästhesiegerät ausgestaltet sein, welches der dem Patienten zugeführten Atemgas ein Narkosemittel zusetzt. Optional wird die ausgeatmete Luft, die in einer Ausgestaltung Narkosemittel enthalten kann, wieder dem Beatmungsgerät zugeführt, so dass ein Beatmungskreislauf erzeugt wird.

Um die künstliche Beatmung soweit wie gewünscht automatisiert durchführen zu können, ist es erforderlich, die Konzentrationen von mehreren Gasen im Beatmungskreislauf zu messen, insbesondere die Konzentrationen von $O_2$ und $CO_2$ sowie optional von $N_2O$ (Lachgas) und / oder mindestens eines Narkosemittels. In der Regel sind diese Gaskonzentrationen zeitlich veränderlich. Insbesondere ist bei einem Einatmungsvorgang (Inspiration) die Konzentration an $O_2$ größer als bei einem Ausatmungsvorgang (Exspiration), während bei dem Ausatmungsvorgang die Konzentration an $CO_2$ in der Regel größer ist als bei dem Einatmungsvorgang. Eine Ausnahme kann auftreten, wenn bei einer künstlichen Beatmung des Patienten die Konzentration einer Komponente des Gasgemisches, welches dem Patienten zugeführt wird, rasch verringert wird. Diese Komponente ist beispielsweise Sauerstoff oder ein Atemgas.

Eine häufig und auch erfindungsgemäß angewendete Möglichkeit, um die Gaskonzentrationen zu messen, ist die folgende: Eine Gasprobe wird an einem Abzweigpunkt nahe dem Patienten aus dem Beatmungskreislauf abgesaugt, einer Gassensor-Anordnung zugeführt und anschließend wieder in den Beatmungskreislauf eingespeist. Dieser Abzweigpunkt ist beispielsweise in einem Y-Stück nahe dem Patienten realisiert. Die Gassensor-Anordnung misst die gesuchte Gaskonzentrationen in der abgezweigten Gasprobe. Eine solche Vorgehensweise wird beispielsweise in DE 10 2017 011 625 A1 beschrieben.

Zwischen dem Abzweigpunkt und der Gassensor-Anordnung kann eine Leckage auftreten, beispielsweise weil Bestandteile, die für die künstliche Beatmung oder die Gasmessung verwendet werden, aufgrund eines Fehlers nicht fluiddicht miteinander verbunden sind. Durch eine solche Leckage hindurch kann Umgebungsluft eingesaugt werden. Die eingesaugte Umgebungsluft kann Messergebnisse der Gassensor-Anordnung verfälschen. Daher muss unverzüglich erkannt werden, damit die Leckage beseitigt wird oder die Beatmung ohne die Ergebnisse der Gasmessung fortgesetzt wird. Eine Leckage muss insbesondere dann unverzüglich erkannt werden, wenn mindestens ein Parameter der künstlichen Beatmung abhängig von der gemessenen Konzentration mindestens eines Gases automatisch geregelt wird oder ein Anwender diesen Parameter abhängig von der gemessenen Gas-Konzentration einstellt oder verändert.

Mehrere Verfahren und Vorrichtungen sind bekannt geworden, um während der künstlichen Beatmung eines Patienten automatisch eine Leckage in einer Fluidverbindung zu einer Gassensor-Anordnung zu erkennen.

In DE 10 2017 011 625 A1 wird vorgeschlagen, den zeitlichen Verlauf von $CO_2$ und den zeitlichen Verlauf eines anderen Gases, insbesondere von $O_2$, zu messen und die beiden zeitlichen Verläufe miteinander zu vergleichen. Falls keine Leckage aufgetreten ist, so sind die beiden Verläufe relativ zueinander phasenverschoben. Ein statistisches Maß für die Phasenverschiebung zwischen den beiden Verläufen, insbesondere die Covarianz, wird berechnet und mit einer vorgegebenen Schranke verglichen. Falls keine Leckage aufgetreten ist, so beträgt diese Covarianz idealerweise -1 und liegt in der Praxis zwischen -0,6 und -0,8. In dieser Druckschrift wird auch eine Kovarianz zwischen einem ersten und einem zweiten zeitlichen Verlauf von Konzentrationsänderungen als einer Leckage-Erkennung zugrundeliegendes Ähnlichkeitsmaß offenbart.

In US 8,033,280 B2 und EP 1961439 A1 wird vorgeschlagen, den jeweiligen zeitlichen Verlauf von zwei Gasen zu messen. Falls diese beiden zeitlichen Verläufe sich gleichzeitig in Richtung von ebenfalls gemessenen oder bekannten Gaskonzentrationen in der Umgebungsluft bewegen, so ist eine Leckage erkannt.

In WO 2004/076944 A2 wird vorgeschlagen, den zeitlichen Verlauf eines Drucks in einem Patienten-Monitor und den zeitlichen Verlauf eines Drucks in einem Beatmungskreislauf zu messen und diese beiden zeitlichen Verläufe miteinander zu vergleichen.

Der Erfindung liegt die Aufgabe zugrunde, ein Verfahren zur Überwachung eines Mess-Systems bereitzustellen, wobei das Mess-System für eine künstliche Beatmung eines Patienten verwendet wird und eine Gasprobe aus einer Fluidverbindung zwischen dem Patienten und einem medizinischen Gerät absaugt. Das Verfahren soll automatisch und mit höherer Zuverlässigkeit als bekannte Verfahren eine Leckage detektieren, wobei die zu detektierende Leckage eine Messung der Konzentration mindestens eines Gases in der abgesaugten Gasprobe beeinträchtigen kann. Weiterhin liegt der Erfindung die Aufgabe zugrunde, ein derartiges Mess-System bereitzustellen.

Die Erfindung wird durch ein Verfahren mit den Merkmalen des Anspruchs 1 sowie durch ein Mess-System mit den Merkmalen des Anspruchs 9 gelöst. Vorteilhafte Ausgestaltungen sind in den Unteransprüchen angegeben. Vorteilhafte Ausgestaltungen des erfindungsgemäßen Verfahrens sind, wo sinnvoll, auch vorteilhafte Ausgestaltungen des erfindungsgemäßen Mess-Systems und umgekehrt.

Das erfindungsgemäße Verfahren überwacht ein Mess-System. Dieses Mess-System lässt sich für die künstliche Beatmung eines Patienten verwenden. Das Verfahren zur Überwachung wird durchgeführt, während der Patient künstlich beatmet wird. Der Patient kann während der künstlichen Beatmung vollständig sediert sein. Das erfindungsgemäße Verfahren lässt sich auch dann anwenden, wenn die künstliche Beatmung eine spontane Atmung (eigene Atmungsaktivität) des Patienten überlagert.

Das Mess-System umfasst eine Gassensor-Anordnung und eine Gassensor-Fluidführungseinheit. Eine Fluidführungseinheit im Sinne der Ansprüche vermag ein Fluid von einem Punkt zu einem anderen Punkt zu führen, idealerweise ohne dass auf dem Weg zwischen diesen beiden Punkten Fluid entweicht oder ein Gas aus der Umgebung in das Innere der Fluidführungseinheit eindringen kann. Die Fluidführungseinheit umfasst nicht notwendigerweise eine Fördereinheit. Die Fluidführungseinheit ist insbesondere ein Schlauch, kann aber auch ein Rohr oder eine Kombination aus Schlauch und Rohr sein. Eine Leckage ist aufgetreten, wenn Gas in die Fluidführungseinheit eindringen kann und / oder Gas aus der Fluidführungseinheit austreten kann.

Das Verfahren wird durchgeführt, während eine Fluidverbindung zwischen dem Patienten und einem medizinischen Gerät hergestellt ist. Diese Fluidverbindung wird mithilfe einer Patienten-Fluidführungseinheit hergestellt. Das medizinische Gerät ist insbesondere ein Beatmungsgerät oder ein Anästhesiegerät oder ein Patienten-Monitor. Die Patienten-Fluidführungseinheit verbindet den Patienten mit dem medizinischen Gerät.

Das erfindungsgemäße Verfahren umfasst die folgenden Schritte, die automatisch durchgeführt werden: Eine Gasprobe wird aus der Patienten-Fluidführungseinheit abgesaugt.

Die abgesaugte Gasprobe wird durch die Gassensor-Fluidführungseinheit hindurch zu der Gassensor-Anordnung geleitet.

Ein Maß für den zeitlichen Verlauf der Konzentration von Kohlendioxid ($CO_2$) in der abgesaugten Gasprobe wird berechnet.

Ein Maß für den zeitlichen Verlauf der Konzentration eines weiteren Gases in der abgesaugten Gasprobe wird berechnet. Dieses weitere Gas ist von $CO_2$ verschieden und enthält bevorzugt Sauerstoff ($O_2$).

Die beiden zeitlichen Konzentrations-Verläufe werden unter Verwendung von Messwerten berechnet, welche die Gassensor-Anordnung abhängig von der abgesaugten Gasprobe erzeugt hat.

Ein Maß für einen zeitlichen Verlauf der zeitlichen Veränderung der $CO_2$-Konzentration wird berechnet. Die zeitliche Veränderung einer Größe ist die Ableitung der Größe nach der Zeit.

Ein Maß für einen zeitlichen Verlauf der zeitlichen Veränderung der Konzentration des weiteren Gases, insbesondere für den zeitlichen Verlauf der zeitlichen Veränderung der $O_2$-Konzentration, wird berechnet.

Um diese beiden zeitlichen Verläufe der beiden zeitlichen Veränderungen zu berechnen, werden die beiden zeitlichen Konzentrations-Verläufe verwendet.

Gesucht wird nach jeder vorzeichengleichen Zeitspanne. Eine vorzeichengleiche Zeitspanne ist eine Zeitspanne, in der die beiden Konzentrations-Veränderungs-Verläufe durchgehend dasselbe Vorzeichen aufweisen, also eine Zeitspanne, in der die Konzentrations-Veränderungs-Verläufe beide größer null oder beide kleiner null sind.

Dann, wenn mindestens eine solche vorzeichengleiche Zeitspanne detektiert worden ist, werden die folgenden Schritte durchgeführt:

- Geprüft wird, ob die beiden Konzentrations-Veränderungs-Verläufe ein vorgegebenes erstes Leckage-Kriterium erfüllen, und zwar in der oder einer oder jeder detektierten vorzeichengleichen Zeitspanne.
- Dann, wenn das erste Leckage-Kriterium in mindestens einer geprüften vorzeichengleichen Zeitspanne erfüllt ist, wird entschieden, dass eine Leckage zwischen der Patienten-Fluidführungseinheit und der Gassensor-Anordnung aufgetreten ist. Mindestens wird in diesem Fall entschieden, dass ein Indiz für eine solches Leckage aufgetreten ist, und dann wird bevorzugt eine weitere automatische Prüfung durchgeführt oder eine Prüfung ausgelöst oder ein Alarm ausgegeben.

Das erfindungsgemäße Mess-System umfasst die gerade beschriebene Gassensor-Anordnung, die gerade beschriebene Gassensor-Fluidführungseinheit und eine Signalverarbeitungseinheit. Diese Signalverarbeitungseinheit ist dazu ausgestaltet,

- die Konzentrations-Verläufe und die Konzentrations-Veränderungs-Verläufe zu berechnen,
- nach vorzeichengleichen Zeitspannen zu suchen,
- zu entscheiden, ob das erste Leckage-Kriterium erfüllt ist, und
- anschließend zu entscheiden, ob eine Leckage oder mindestens ein Indiz für eine Leckage aufgetreten sind.

Die Signalverarbeitungseinheit ist also dazu ausgestaltet, automatisch diejenigen Schritte des erfindungsgemäßen Verfahrens durchzuführen, bei denen jeweils Messwerte ausgewertet werden.

Erfindungsgemäß wird eine Gasprobe aus der Patienten-Fluidführungseinheit abgesaugt und durch die Gassensor-Fluidführungseinheit zu der Gassensor-Anordnung geleitet. Diese Ausgestaltung ermöglicht es, einerseits die Gasprobe an einem Messpunkt, der nahe dem Patienten angeordnet ist, abzusaugen. Andererseits lässt sich dank der Gassensor-Fluidführungseinheit die Gassensor-Anordnung räumlich entfernt vom Patienten, beispielsweise in dem medizinischen Gerät oder einem Patienten-Monitor, anordnen.

Das Mess-System steht in einer Fluidverbindung mit der Patienten-Fluidführungseinheit und ist dann, wenn keine Leckage aufgetreten ist, fluiddicht gegen die Umgebungsluft abgedichtet. Der Erfindung liegt die Erkenntnis zugrunde, dass bei einem leckfreien Zustand des Mess-Systems der zeitliche Verlauf der $CO_2$-Konzentration gegenläufig zum zeitlichen Verlauf der Konzentration des weiteren Gases ist. Daher haben die zeitlichen Verläufe der beiden zeitlichen Veränderungen dieser Konzentrationen stets das entgegengesetzte Vorzeichen, falls keine Leckage aufgetreten ist und keine sonstige Störgröße die zeitlichen Verläufe signifikant beeinflusst. Eine Leckage stellt hingegen eine Fluidverbindung zwischen dem Mess-System und der Umgebung her. Falls eine vorzeichengleiche Zeitspanne der zeitlichen Änderungen der $CO_2$- Konzentration und der weiteren Konzentration entdeckt wird, so ist dies erfindungsgemäß ein Indiz dafür, dass eine Leckage aufgetreten ist.

Erfindungsgemäß hängt das erste Leckage-Kriterium von den beiden Konzentrations-Veränderungs-Verläufen in der oder jeder detektierten vorzeichengleichen Zeitspanne ab. Dieses erste Leckage-Kriterium hängt deutlich weniger, idealerweise überhaupt nicht, von einem Absolutwert einer Gaskonzentration oder eines Gasdrucks ab. Dadurch ist dieses erste Leckage-Kriterium weniger empfindlich als andere mögliche Leckage-Kriterien gegenüber Einstellungen bei der künstlichen Beatmung und Störgrößen und liefert daher in vielen Fällen bei annähernd gleicher Zuverlässigkeit weniger Fehlalarme.

Das erste Leckage-Kriterium lässt sich so ausgestalten, dass einerseits mit ausreichend hoher Zuverlässigkeit jede Leckage - oder wenigstens jede Leckage, welches die Messung der Gaskonzentrationen erheblich beeinflussen könnte - detektiert wird und andererseits nur relativ wenige Fehlalarme erzeugt werden.

Das Merkmal, dass abhängig von den beiden Konzentrations-Veränderungs-Verläufen vorzeichengleiche Zeitspannen detektiert werden, erspart die Notwendigkeit, eine Leckage abhängig von einem Vergleich eines berechneten Parameterwerts mit einem vorgegebenen Schwellwert oder einem zuvor berechneten Referenz-Parameterwert zu detektieren. Vielmehr basiert das erste Leckage-Kriterium auf zeitlichen Veränderungen von Gaskonzentrationen. Insbesondere deswegen lässt sich durch Anwendung des erfindungsgemäßen erste Leckage-Kriterium eine Leckage auch dann mit relativ hoher Zuverlässigkeit erkennen, wenn diese Leckage nicht plötzlich, sondern allmählich auftritt, beispielsweise aufgrund einer Materialermüdung. Gerade eine solche allmählich auftretende Leckage lässt sich oft nicht durch eine optische Inspektion und auch bei Anwendung anderer möglicher Leckage-Kriterien oft nicht automatisch detektieren.

Außerdem setzen das erfindungsgemäße Verfahren und das erfindungsgemäße Mess-System nicht voraus, dass Referenz-Messwerte bei einem garantiert leckfreien Zustand vorliegen, um dann aktuelle Messwerte mit diesen beim leckfreien Zustand gewonnenen Referenz-Messwerten vergleichen zu können. Die Erfindung vermag vielmehr auch dann eine Leckage zu detektieren, wenn die Leckage bereits bei Beginn der Messung vorliegt, beispielsweise weil Bestandteile eines Beatmungskreislaufs falsch und daher nicht fluiddicht miteinander verbunden sind oder weil die Materialermüdung bereits bei Beginn der Messung vorliegt.

Die Erfindung lässt sich in vielen Fällen in ein bereits vorhandenes Mess-System integrieren, bevorzugt indem auf einer Signalverarbeitungseinheit des Mess-Systems geeignete Software implementiert wird. Die benötigten Sensoren für die $CO_2$-Konzentration und für die Konzentration des weiteren Gases sowie optional ein Sensor für den Druck in der Gassensor-Anordnung und / oder ein Sensor für den Umgebungsdruck sind in der Regel bereits vorhanden, oft auch die Signalverarbeitungseinheit. Die Erfindung benötigt keine weiteren Sensoren.

Die Erfindung lässt sich in Kombination mit einer Ausgestaltung einsetzen, bei welcher die Gassensor-Fluidführungseinheit zeitweise von der Patienten-Fluidführungseinheit getrennt wird, sodass keine Gasprobe von der Patienten-Fluidführungseinheit zu der Gassensor-Anordnung fließen kann, und bei welcher in diesem Zustand nach einer Leckage gesucht wird. Die Erfindung setzt aber nicht voraus, dass die Gassensor-Fluidführungseinheit zeitweise getrennt wird, um nach einer Leckage zu suchen. Daher können die beiden Vorgänge, Gaskonzentrationen zu messen und das Mess-System auf eine Leckage zu überwachen, zeitlich überlappend und insbesondere kontinuierlich ohne Unterbrechung durchgeführt werden. Daher lässt sich die erfindungsgemäße automatische Überwachung auf Leckagen durchführen, während ein Patient künstlich beatmet wird.

Messwerte der Gassensor-Anordnung werden erfindungsgemäß dafür verwendet, um die zeitlichen Verläufe von $CO_2$ und mindestens einem weiteren Gas zu berechnen und unter Verwendung der zeitlichen Verläufe zu entscheiden, ob eine Leckage vorliegt oder nicht. Möglich ist, dass die zeitlichen Verläufe zusätzlich für die künstliche Beatmung des Patienten verwendet werden, z.B. um ein Beatmungsgerät anzusteuern und / oder um die spontane Atmung des Patienten zu messen. Möglich ist, dass die zeitlichen Verläufe von $CO_2$ und mindestens zwei weiteren Gasen berechnet werden, z.B. von $O_2$, $N_2O$ und mindestens einem Narkosemittel.

Unterschiedliche Parameter, von denen das erste Leckage-Kriterium abhängt, lassen sich vorgeben. Diese Parameter lassen sich so vorgeben, dass die Anzahl von Fehlalarmen relativ gering bleibt und trotzdem eine tatsächlich aufgetretene Leckage mit hoher Zuverlässigkeit entdeckt wird.

In der Regel ist die CO2-Konzentration in dem ausgeatmeten Atemgas größer als die CO2-Konzentration in dem eingeatmeten und / oder von einem Beatmungsgerät oder Anästhesiegerät zugeführten Atemgas. In einer Ausgestaltung wird daher jede Zeitspanne detektiert, in welcher der Patient Atemgas ausatmet. Verglichen mit anderen Zeitspannen führt daher während einer solchen Exspirations-Zeitspanne eine Leckage in der Regel zu einer größeren Reduktion der CO2-Konzentration. Bevorzugt wird nur in einer solchen Exspirations-Zeitspanne nach vorzeichengleichen Zeitspannen gesucht. Verglichen mit einer Ausgestaltung, bei der stets nach vorzeichengleichen Zeitspannen gesucht wird, spart diese Ausgestaltung Rechenzeit und / oder Rechenkapazität ein. Weil außerhalb einer Exspirations-Zeitspanne kein vermeintliche Leckage detektiert werden kann, wird die Gefahr von Fehlalarmen reduziert.

Erfindungsgemäß werden der zeitliche Verlauf der CO2-Konzentration sowie der zeitliche Verlauf der Konzentration des weiteren Gases in der abgesaugten Gasprobe gemessen. Dieses weitere Gas ist von CO2 verschieden und enthält insbesondere Sauerstoff (O2), Lachgas (N2O) oder ein Narkosemittel. Es ist möglich, das Verfahren auf die Konzentrationen von drei verschiedenen Gasen auszudehnen.

In einer Ausgestaltung wird eine Leckage detektiert, wenn ein zweites vorgegebenes Leckage-Kriterium erfüllt ist. Das zweite Leckage-Kriterium hängt einerseits von der zeitlichen Veränderung eines Maßes für die Phasenverschiebung zwischen den beiden zeitlichen Konzentrations-Verläufen und andererseits von der zeitlichen Veränderung eines Drucks in oder flussaufwärts von der Gassensor-Anordnung ab. Das Maß für die Phasenverschiebung wird bevorzugt durch Anwendung eines statistischen Verfahrens berechnet, besonders bevorzugt als eine Covarianz. Auch das zweite Leckage-Kriterium erfordert nicht, dass ein gemessener Parameterwert mit einem Schwellwert oder mit einem zuvor gemessenen Referenz-Parameterwert verglichen wird.

Dem zweiten Leckage-Kriterium liegt die Erkenntnis zugrunde, dass eine Leckage, insbesondere ein plötzlich auftretende Leckage, in der Regel sowohl zu einer Veränderung der Phasenverschiebung als auch zu einer Veränderung des Drucks im Mess-System führt, insbesondere zu einer Veränderung des gemessenen Drucks relativ zum Umgebungsdruck. Das zweite Leckage-Kriterium basiert auf diesen beiden Auswirkungen einer Leckage. Das zweite Leckage-Kriterium vermag mit höherer Zuverlässigkeit als andere mögliche Leckage-Kriterien plötzlich auftretende Leckagen zu detektieren.

Möglich ist, ein Verfahren zur Erkennung einer Leckage ausschließlich basierend auf dem zweiten Leckage-Kriterium durchzuführen, wobei eine Gasprobe abgesaugt wird und wobei das Maß für die Phasenverschiebung sowie die zeitliche Veränderung des Druckverlaufs gemessen werden, wobei aber nicht notwendigerweise nach vorzeichengleichen Zeitspannen gesucht wird und wobei nicht notwendigerweise das erste Leckage-Kriterium verwendet wird.

In einer bevorzugten Ausgestaltung wird ein mittlerer Druck im Mess-System ermittelt, beispielsweise gemittelt über eine komplette Atemphase (Atemzug) oder eine Einatemphase oder eine Ausatemphase des Patienten. Die zeitliche Veränderung dieses mittleren Drucks wird für das zweite Leckage-Kriterium verwendet. Indem ein gemittelter Druck verwendet wird, werden zufällige Schwankungen des Drucks rechnerisch eliminiert. Eine Leckage bewirkt hingegen eine systematische Druckänderung, die zu einer zeitlichen Veränderung des mittleren Drucks führt.

In einer bevorzugten Ausgestaltung wird hingegen eine Leckage - oder ein Indiz für eine Leckage - dann detektiert, wenn das erste Leckage-Kriterium, das zweite Leckage-Kriterium oder beide Leckage-Kriterien erfüllt sind. Diese Ausgestaltung ermöglicht es, sowohl eine sich langsam entwickelnde Leckage durch das erste Leckage-Kriterium als auch eine plötzlich auftretende Leckage durch das zweite Leckage-Kriterium mit relativ hoher Zuverlässigkeit zu detektieren. In vielen Fällen führt eine plötzlich auftretende Leckage dazu, dass beide Leckage-Kriterien erfüllt sind.

Bei Verwendung des zweiten Leckage-Kriteriums wird bevorzugt eine Zeitverschiebung vorgegeben. Diese Zeitverschiebung hängt in einer Ausgestaltung von dem Abstand zwischen dem Messpunkt, an dem die Gaskonzentrationen gemessen werden, und dem Messpunkt, an dem der Druck gemessen wird, ab, optional zusätzlich von der Fließgeschwindigkeit eines Gases, während das Gas von dem einen zu dem anderen Messpunkt fließt. Diese vorgegebene Zeitverschiebung berücksichtigt folgende Tatsache: Wenn die Gaskonzentrationen und der Druck an unterschiedlichen Messpunkten gemessen werden, führt eine plötzlich auftretende Leckage zu einem Zeitverzug zwischen der Veränderung des Drucks und der Veränderung der Phasenverschiebung.

In einer anderen Ausgestaltung hängt diese Zeitverschiebung von der Phasenverschiebung zwischen dem Verlauf der CO2-Konzentration und dem Verlauf der Konzentration des weiteren Gases ab.

Wie bereits dargelegt, betrifft die Erfindung ein Mess-System, das für die künstliche Beatmung eines Patienten verwendbar ist. In einer Ausgestaltung ist dieses Mess-System ein Bestandteil einer Verbindungs-Anordnung, welche außerdem eine Patienten-Fluidführungseinheit umfasst. Mithilfe dieser Patienten-Fluidführungseinheit lässt sich eine Fluidverbindung zwischen einem Patienten und einem medizinischen Gerät herstellen. Wenigstens zeitweise ist die Gassensor-Fluidführungseinheit des erfindungsgemäßen Mess-Systems mit der Patienten-Fluidführungseinheit verbunden.

Bevorzugt erzeugt das Mess-System einen Alarm und übermittelt diesen Alarm an einen räumlich entfernten Empfänger und / oder gibt diesen Alarm in einer für einen Menschen wahrnehmbaren Form aus, wenn eine Leckage detektiert ist. In einer Ausgestaltung erzeugt das Mess-System den Alarm bereits dann, wenn das erste Leckage-Kriterium und / oder das zweite Leckage-Kriterium erfüllt sind.

In einer anderen Ausgestaltung führt das Mess-System dann, wenn das erste oder optional mindestens ein Leckage-Kriterium erfüllt ist, zunächst eine Überprüfung aus. Für diese Überprüfung wird der Schritt, eine Gasprobe aus der Patienten-Fluidführungseinheit abzusaugen, vorübergehend unterbrochen. Durch die Patienten-Fluidführungseinheit fließt hingegen bevorzugt weiterhin Gas, weil die künstliche Beatmung des Patienten fortgesetzt wird. Während dieser Unterbrechung werden der Druck in oder an dem Mess-System sowie der Druck in der Patienten-Fluidführungseinheit jeweils mindestens einmal gemessen, und die gemessenen Drücke werden miteinander verglichen. Falls tatsächlich eine Leckage aufgetreten ist, so weicht der Druck in oder an dem Mess-System signifikant von dem Druck in der Patienten-Fluidführungseinheit ab. Mindestens in einer Inspirations-Zeitspanne ist der Druck in dem Mess-System aufgrund einer Leckage in der Regel kleiner als in der Patienten-Fluidführungseinheit. In diesem Falle wird ein Alarm generiert. Durch diese Überprüfung lässt sich die Anzahl von Fehlalarmen weiter reduzieren, wobei gleichzeitig sichergestellt ist, dass in der Regel eine tatsächlich auftretende Leckage detektiert wird.

In einer weiteren Ausgestaltung betrifft die Erfindung ein medizinisches System. Dieses medizinische System umfasst ein medizinisches Gerät, insbesondere ein Beatmungsgerät oder ein Anästhesiegerät oder einen Patienten-Monitor, sowie die gerade beschriebene Verbindungs-Anordnung mit dem erfindungsgemäßen Mess-System. Die Verbindungs-Anordnung vermag wenigstens zeitweise eine Fluidverbindung zwischen dem medizinischen Gerät und einem Patienten herzustellen.

Bevorzugt vermag das medizinische Gerät den zeitlichen Verlauf der CO2-Konzentration und den zeitlichen Verlauf des oder mindestens eines weiteren Gases zu empfangen. Diese mindestens zwei zeitlichen Verläufe wurden von der Signalverarbeitungseinheit des Mess-Systems berechnet, wofür die Signalverarbeitungseinheit Messwerte von der Gassensor-Anordnung verwendet. Das medizinische System vermag in einer Ausgestaltung die empfangenen zeitlichen Verläufe automatisch zu verarbeiten, beispielsweise um einen Aktuator anzusteuern, welcher Beatmungshübe erzeugt. In einer Ausgestaltung vermag das medizinische Gerät die empfangenen zeitlichen Verläufe in einer von einem Menschen wahrnehmbaren Form auszugeben, bevorzugt grafisch auf einer Ausgabeeinheit.

In einer Ausgestaltung ist die Signalverarbeitungseinheit des erfindungsgemäßen Mess-Systems ein Bestandteil des medizinischen Geräts. Sie kann auch räumlich vom medizinischen Gerät getrennt sein.

Im Folgenden wird die Erfindung anhand eines Ausführungsbeispiels beschrieben. Hierbei zeigen

Figur 1    einen Beatmungskreislauf zur künstlichen Beatmung eines Patienten;

Figur 2    den zeitlichen Verlauf der CO2- Konzentration und den zeitlichen Verlauf der O2-Konzentration während eines einzigen Atemzugs bei Abwesenheit einer Leckage;

Figur 3    die zeitlichen Verläufe von Figur 2 bei Anwesenheit einer Leckage;

Figur 4    die skalierten zeitlichen Verläufe von CO2, O2, N2O und Narkosemittel bei Abwesenheit und bei Anwesenheit einer Leckage;

Figur 5    die auf einen Bereich von -1000 bis +1000 normierten zeitlichen CO2- und O2-Verläufe von Figur 2 und Figur 3;

Figur 6    die zeitlichen Veränderungen der normierten zeitlichen CO2- und O2-Verläufe von Figur 5 sowie den zeitlichen Verlauf der Produktwerte;

Figur 7    Auswirkungen von Leckagen, die in einem Versuch mehrmals erzeugt und wieder beseitigt wurden, und deren Erkennung;

Figur 8    den zeitlichen Verlauf der CO2-Konzentration und den der Covarianz zwischen CO2 und O2;

Figur 9    den zeitlichen Verlauf des Drucks $P_{Cell}$ sowie des Signals DiPhaC bei einer Beatmung mit hoher Frequenz und niedrigem Beatmungsdruck;

Figur 10    beispielhaft die zeitlichen Verläufe von $\Delta Cov[CO2,O2]$ und $\Delta P_{Cell}$;

Figur 11    in der Situation von Figur 9 den zeitlichen Verlauf des Signals DPC.

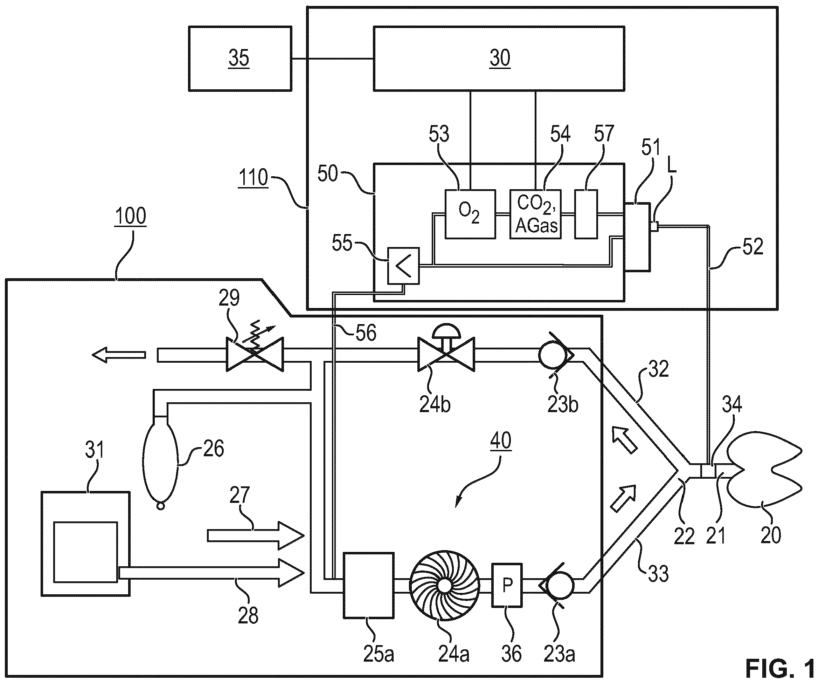

Figur 1 zeigt schematisch einen Beatmungskreislauf 40, um einen Patienten 20 künstlich zu beatmen. Möglich ist, dass der Beatmungskreislauf 40 dem Patienten 20 ein Narkosemittel zuführt, sodass der Patient 20 teilweise oder vollständig sediert ist. Die Erfindung lässt sich auch in einem Beatmungskreislauf 40 einsetzen, in dem der Patient 20 zwar künstlich beatmet wird, ihm aber kein Narkosemittel zugeführt wird. Möglich ist, dass die künstliche Beatmung und die spontane Atmung des Patienten 20 sich überlagern.

Eine patientenseitige Koppeleinheit 21, beispielsweise ein Mundstück oder eine Atemmaske, verbindet den Patienten 20 mit dem Beatmungskreislauf 40. Das Mundstück 21 ist mit einem Y-Stück 22 verbunden. Das Y-Stück 22 ist mit einer Atemgasleitung 32 für die Einatmung (Inspiration) und mit einer Atemgasleitung 33 für die Ausatmung (Exspiration) verbunden.

Der Beatmungskreislauf 40 verbindet die patientenseitige Koppeleinheit 21 mit dem Beatmungsgerät 100, welches in einer Ausgestaltung des Ausführungsbeispiels als Anästhesiegerät ausgestaltet ist. Der Beatmungskreislauf 40 ist durch das Beatmungsgerät 100 hindurchgeführt.

Figur 1 zeigt schematisch, welche nachfolgend beschriebenen Bestandteile zum Beatmungsgerät 100 des Ausführungsbeispiels gehören.

Eine Förderereinheit 24a in Form einer Pumpe oder eines Gebläses saugt Atemgas an und erzeugt einen permanenten Strom von Atemgas durch die Einatmungs-Atemgasleitung 32 auf das Y-Stück 22 und auf den Patienten 20 zu. Bevorzugt arbeitet diese Förderereinheit 24a als ein Verdichter, der einen Überdruck erzeugt und mit einer Drehzahl von über 10.000 Umdrehungen/min rotiert. Ein Kohlendioxidabsorber 25a vermag Kohlendioxid aus dem Beatmungskreislauf 40 zu absorbieren. Ein Rückschlagventil 23a lässt einen Gasfluss in der Einatmungs-Atemgasleitung 32 auf das Y-Stück 22 zu passieren und sperrt einen Gasfluss in umgekehrter Richtung.

Ein Rückschlagventil 23b lässt einen Gasfluss in der Ausatmungs-Atemgasleitung 33 vom Y-Stück 22 weg passieren und sperrt einen Gasfluss in umgekehrter Richtung. Ein ansteuerbares PEEP-Ventil 24b (PEEP = "positive end-expiratory pressure") lässt je nach Stellung den Luftstrom, den die Förderereinheit 24a erzeugt hat, passieren oder sperrt ihn und erzeugt dadurch die einzelnen Beatmungshübe und legt die Amplituden und Frequenzen dieser Beatmungshübe fest. Das PEEP-Ventil 24b stellt außerdem sicher, dass auch am Ende der Ausatmung oder bei einer kurzfristigen Öffnung oder Unterbrechung des Beatmungskreislaufs 40 ein ausreichend großer Luftdruck in der Lunge des Patienten 20 aufrechterhalten bleibt. Ein Überdruckventil 29 vermag einen Überdruck in dem Beatmungskreislauf 40 abzubauen, indem Atemgas in die Umgebung abgegeben wird. Dieses Überdruckventil 29 ist bevorzugt als ein "adjustable pressure limiting valve" ausgestaltet und reduziert die Gefahr, dass die Lunge des Patienten 20 geschädigt wird, insbesondere bei einer manuellen Beatmung durch einen Atembeutel 26. Die Druckschranke, bei der dieses Überdruckventil 29 öffnet, lässt sich manuell von außen und / oder automatisch durch eine Ansteuerung des Überdruckventils 29 einstellen.

Ein optionaler Narkosemittelverdampfer 31 vermag einen Fluidstrom 28 mit einer dampfförmigen Mischung aus einem Trägergas und mindestens einem Narkosemittel in den Beatmungskreislauf 40 einzuspeisen. Außerdem lässt sich dem Beatmungskreislauf 40 ein Fluidstrom 27 von Frischluft oder einem sonstigen frischen Gas zuführen.

Der Beatmungskreislauf 40 wird von der Förderereinheit 24a und optional von einem Atembeutel 26, der manuell betätigt werden kann, in Gang gehalten.

Die Förderereinheit 24a, der optionale Narkosemittelverdampfer 31, der Kohlendioxidabsorber 25a, die Rückschlagventile 23a und 23b sowie die Ventile 24b und 29 gehören zu dem schematisch gezeigten Beatmungsgerät 100, welches als Anästhesiegerät ausgestaltet sein kann. Möglich ist auch, dass der Beatmungskreislauf 40 ausschließlich mithilfe des Atembeutels 26 im Gang gehalten wird, beispielsweise an Bord eines Fahrzeugs oder an einem anderen Ort, an dem keine stationäre Stromversorgung zur Verfügung steht oder die Stromversorgung oder die Förderereinheit 24a ausgefallen sind.

Ein Steuergerät 35 empfängt Messwerte von einem Drucksensor 58, der den Luftdruck $P_{amb}$ in der Umgebung des Beatmungsgeräts 100 misst. Außerdem empfängt das Steuergerät 35 Messwerte von einem Drucksensor 36, der den aktuellen Druck im Beatmungskreislauf 40 misst, beispielsweise den am Patienten 20 anliegenden Beatmungsdruck (airway pressure, $P_{aw}$), bevorzugt als Druck relativ zum Umgebungsdruck $P_{amb}$. Das Steuergerät 35 steuert die Förderereinheit 24a, den Narkosemittelverdampfer 31 und weitere Bestandteile des Beatmungsgeräts 100 an, um eine gewünschte Beatmung des Patienten 20 zu realisieren.

Für die Ansteuerung des Beatmungsgeräts 100 ist es erforderlich, dass die tatsächliche aktuelle Konzentration von Kohlendioxid (CO2), Sauerstoff (O2), Lachgas (N2O) und optional von eingespeistem Narkosemittel gemessen wird, und zwar die Konzentrationen nahe der patientenseitigen Koppeleinheit 21 und somit nahe dem Mund und / oder der Nase des Patienten 20.

Zu diesem Zweck wird eine Probe mit Atemgas über eine Gasprobe-Fluidführungseinheit in Form eines Entnahmeschlauchs 52 aus dem Beatmungskreislauf 40 entnommen (abgezweigt) und über einen Zuführschlauch 56 wieder in den Beatmungskreislauf 40 eingespeist. Der Entnahmeschlauch 52 beginnt in einem Abzweigpunkt 34 zwischen der patientenseitigen Koppeleinheit 21 und dem Y-Stück 22. Am Abzweigpunkt 34 befindet sich optional ein nicht gezeigtes Ventil, welches in geschlossener Stellung den Entnahmeschlauch 52 vom Beatmungskreislauf 40 abtrennt und welches sich vom Steuergerät 35 ansteuern lässt. Bei geöffnetem Ventil steht der Entnahmeschlauch 52 in einer nicht eingeschränkten Fluidverbindung mit dem Beatmungskreislauf 40. Der Abführschlauch 56 führt zu einem Einspeisepunkt flussaufwärts von dem Kohlendioxidabsorber 25a.

Der Entnahmeschlauch 52 leitet die Atemgasprobe zu einer Gassensor-Anordnung 50. Diese Gassensor-Anordnung 50 ist räumlich von der patientenseitigen Koppeleinheit 21 entfernt und gehört in einer Ausgestaltung zu dem Beatmungsgerät 100. Die Gassensor-Anordnung 50 umfasst eine Pumpe 55, welche Atemgas durch den Entnahmeschlauch 52 ansaugt. Bevorzugt erzeugt die Pumpe 55 an der zum Entnahmeschlauch 52 hin zeigenden Seite dauerhaft einen Unterdruck und an der zum Abführschlauch 56 hin zeigenden Seite dauerhaft einen Überdruck. Die Pumpe 55 vermag einen Volumenstrom von beispielsweise 200 ml/min zu erzeugen.

Ein Sensor 54 vermag Signale zu messen, welche mit der jeweiligen Konzentration von $CO_2$, $N_2O$ und Narkosemittel in der abgesaugten Gasprobe korrelieren. Bevorzugt umfasst dieser Sensor 54 einen Infrarot-Messkopf, der das Dipol-Moment von Molekülen in der Atemgasprobe ausnutzt und die Absorption von infrarot-aktiven Gasen quantitativ auswertet, um die jeweilige Konzentration zu bestimmen. Ein Sensor 53 vermag ein Signal zu messen, welches mit der Konzentration von $O_2$ korreliert. Möglich ist, dass die Gassensor-Anordnung 50 weitere Sensoren umfasst, insbesondere um Redundanz bereitzustellen. Außerdem umfasst die Gassensor-Anordnung 50 einen Drucksensor 57, der den Druck $P_{cell}$ der Atemgasprobe am Eingang der Gassensor-Anordnung 50 misst. Dieser Druck $P_{cell}$ ist zeitlich veränderlich, weil der Druck im Beatmungskreislauf 40 variiert und weil der Entnahmeschlauch 52 mit dem Beatmungskreislauf 40 in einer Fluidverbindung steht, wenn am Abzweigpunkt 34 kein Ventil vorhanden ist oder solange das optionale Ventil am Abzweigpunkt 34 geöffnet ist, sodass der Druck im Beatmungskreislauf 40 sich annähernd mit Schallgeschwindigkeit zu der Gassensor-Anordnung 50 fortpflanzt.

In einer Ausgestaltung messen die Sensoren 53 und 54 Partialdrücke, beispielsweise mit optischen Messverfahren. Der Drucksensor 57 misst den gesamten absoluten Druck $P_{cell,abs}$ der Atemgasprobe. Der Quotient aus einem Partialdruck und dem gesamten absoluten Druck $P_{cell,abs}$ liefert ein Maß für die Konzentration eines Gases in der abgesaugten Gasprobe.

Bevorzugt misst der Drucksensor 57 einen absoluten Druck $P_{cell,abs}$. Im Folgenden wird als Druck $P_{cell}$ der interne Druck in der Gassensor-Anordnung 50 relativ zu dem Umgebungsdruck $P_{amb}$ verwendet, also $P_{cell} = P_{cell,abs} - P_{amb}$. Der relative Druck $P_{cell}$ kann daher auch negative Werte annehmen, nämlich bei einem Unterdruck in der Gassensor-Anordnung 50 relativ zum Umgebungsdruck $P_{amb}$. Bevorzugt wird der relative Druck mehrmals während eines Atmungsvorgangs gemessen. Als Druck $P_{cell}$ wird ein Wert verwendet, der durch geeignete Mittelung über diese während eines Atmungsvorgangs gewonnenen Messwerte berechnet wurde.

Eine datenverarbeitende Signalverarbeitungseinheit 30 empfängt Messwerte von Sensoren der Gassensor-Anordnung 50, insbesondere von dem $CO_2$-, $N_2O$- und Narkosemittelsensor 54, von dem $O_2$-Sensor 53, von den Drucksensoren 57 und 58 und optional von weiteren Sensoren, und wertet diese Messwerte automatisch aus. Die Signalverarbeitungseinheit 30 erzeugt abhängig von den empfangenen Messwerten Signale betreffend die jeweilige aktuelle Konzentration von $O_2$, $CO_2$, $N_2O$ sowie von Narkosemittel und übermittelt diese Signale an das Steuergerät 35. Das Steuergerät 35 verwendet diese empfangenen Signale, um Aktoren des Beatmungsgeräts 100 anzusteuern und dadurch den Beatmungskreislauf 40 automatisch zu regeln.

Die Atemgasprobe, welche bevorzugt kontinuierlich von der Pumpe 55 angesaugt wird, fließt durch eine Wasserfalle 51 hindurch, welche flussaufwärts von den Sensoren 53 und 54 angeordnet ist. Diese Wasserfalle 51 ist mit mindestens einer gasdurchlässigen Membrane ausgestaltet, wobei diese Membrane bevorzugt aus einem chemisch trägen Werkstoff, z.B. Polytetrafluorethylen (PTFE), gefertigt ist. Diese Wasserfalle 51 kann beispielsweise so wie in DE 10 2007 046 533 B3 oder in DE 10 2009 024 040 A1 beschrieben aufgebaut sein. Auf diese Weise wird die angesaugte Atemgasprobe von Kondensat, Partikeln, Schwebstoffen und Keimen befreit. Flüssigkeit, insbesondere auskondensierter Wasserdampf, wird von der Membrane zurückgehalten und fließt in einen Tank der Wasserfalle 51.

Die Gassensor-Anordnung 50, die Wasserfalle 51 und die Signalverarbeitungseinheit 30 gehören zu einem Mess-System 110, das in Figur 1 schematisch gezeigt wird.

Möglich ist, dass auf dem Weg von dem Abzweigpunkt 34 des Entnahmeschlauchs 52 bis zu den Sensoren 54 und 53 eine Leckage auftritt, beispielsweise weil der Entnahmeschlauch 52 nicht fluiddicht mit der patientenseitigen Koppeleinheit 21, dem Y-Stück 22 oder der Wasserfalle 51 verbunden ist oder weil eine Materialermüdung oder eine mechanische Einwirkung von außen zu einer Leckage geführt hat. Diese Leckage kann schlagartig auftreten, beispielsweise weil die künstliche Beatmung begonnen wird, obwohl zwei Teile fälschlicherweise nicht fluiddicht miteinander verbunden sind, oder allmählich, beispielsweise aufgrund von Materialermüdung. In Figur 1 wird beispielhaft eine Leckage L im Übergang zwischen dem Entnahmeschlauch 52 und der Wasserfalle 51 gezeigt.

Eine solche Leckage L kann die Messergebnisse der Sensoren 53 und 54 und von optionalen weiteren Sensoren verfälschen. Denn in dem Entnahmeschlauch 52 tritt wenigstens bei der Exspiration ein Unterdruck relativ zum Umgebungsdruck $P_{amb}$ und auch relativ zum Druck Paw im Beatmungskreislauf 40 auf. Dieser Unterdruck resultiert daraus, dass das Mess-System 110 kontinuierlich eine Atemgasprobe absaugt, und beträgt beispielsweise 100 hPa. Durch diese Leckage L hindurch kann daher Umgebungsluft in den Entnahmeschlauch 52 oder in die Gassensor-Anordnung 50 hinein gesaugt werden. Weil der Unterdruck im Entnahmeschlauch 52 relativ zum Umgebungsdruck $P_{amb}$ variiert, variiert in der Regel auch die Menge der eingesaugten Umgebungsluft über die Zeit.

Die eingesaugte Umgebungsluft kann beispielsweise eine Sauerstoffkonzentration im Beatmungskreislauf 40 vortäuschen, die höher oder auch niedriger als die tatsächliche Sauerstoffkonzentration ist, und daher zu einer falschen Messung führen. Diese falsche Messung kann zu einem Fehler bei der künstlichen Beatmung des Patienten 20 führen. Daher muss eine Leckage L so schnell wie möglich erkannt werden, und ein entsprechender Alarm muss ausgegeben werden, um die Leckage L rasch beseitigen zu können. Andererseits wird gewünscht, möglichst wenige Fehlalarme zu generieren, idealerweise überhaupt keine Fehlalarme.

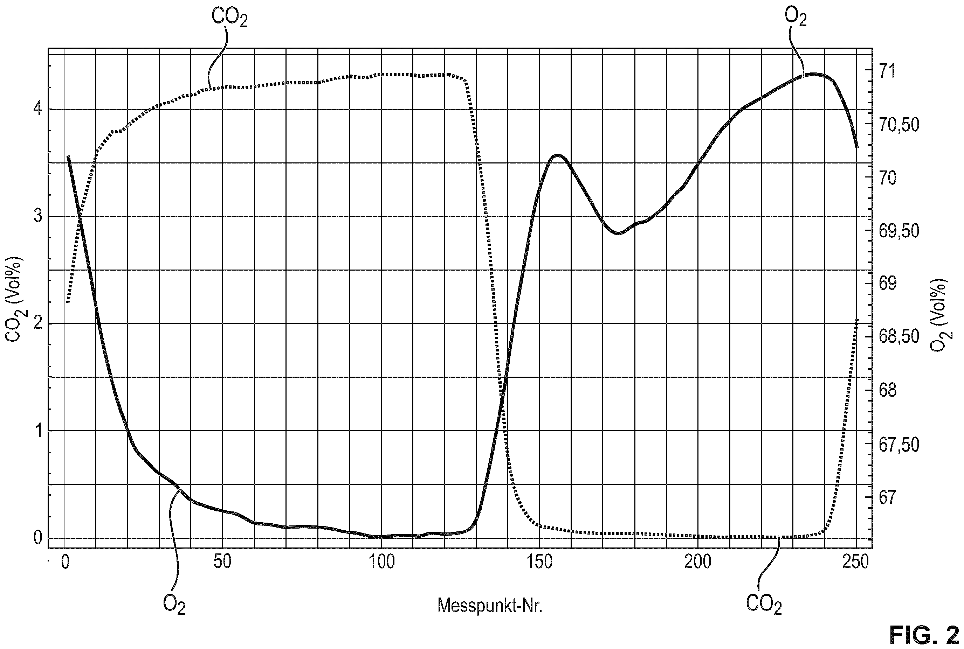

Falls keine Leckage auftritt, so ist der zeitliche Verlauf der O2-Konzentration im Beatmungskreislauf 40 und daher auch in der abgezweigten Atemgasprobe etwa invertiert zu dem zeitlichen Verlauf der CO2-Konzentration, idealerweise phasenverschoben um die halbe Länge eines Atemzugs, falls Einatmung und Ausatmung gleich lange dauern. Während der Einatmung ist die O2-Konzentration deutlich höher, während der Ausatmung in der Regel die CO2-Konzentration. Figur 2 zeigt beispielhaft den zeitlichen Verlauf der O2-Konzentration und den zeitlichen Verlauf der CO2-Konzentration während eines einzelnen Atemzugs in einer Situation, in der keine Leckage aufgetreten ist. Auf der x-Achse ist eine Skala für die Zeit aufgetragen. Die Abtast-Frequenz beträgt also 50 Hz. Die Ziffern bezeichnen die laufenden Nummern der Messpunkte, wobei der Abstand zwischen zwei aufeinanderfolgenden Messpunkten 20 msec beträgt. Die linke y-Achse zeigt eine Skala für die CO2-Konzentration in Volumen-%, die rechte y-Achse eine Skala für die O2-Konzentration in Volumen-%. Im zeitlichen Bereich von 0 sec bis etwa 1,3 sec findet ein Ausatmungsvorgang eines Atemzugs statt, im Bereich von 1,3 sec bis 2,5 sec ein Einatmungsvorgang des nachfolgenden Atemzugs.

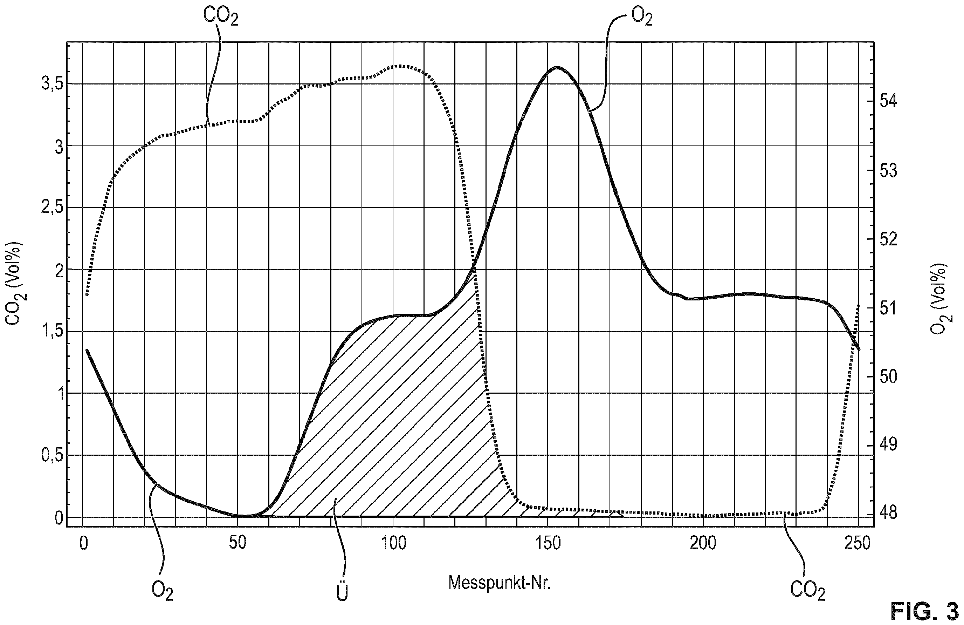

Figur 3 zeigt die beiden zeitlichen Verläufe in der Situation von Figur 2, wobei im gesamten Zeitraum vom 1. bis zum 250. Messpunkt eine Leckage aufgetreten ist. Zu sehen ist, dass die beiden zeitlichen Verläufe von CO2 und O2 nicht mehr gegenläufig sind, sondern eine starke Überlappung im Überlappungsbereich Ü auftritt.

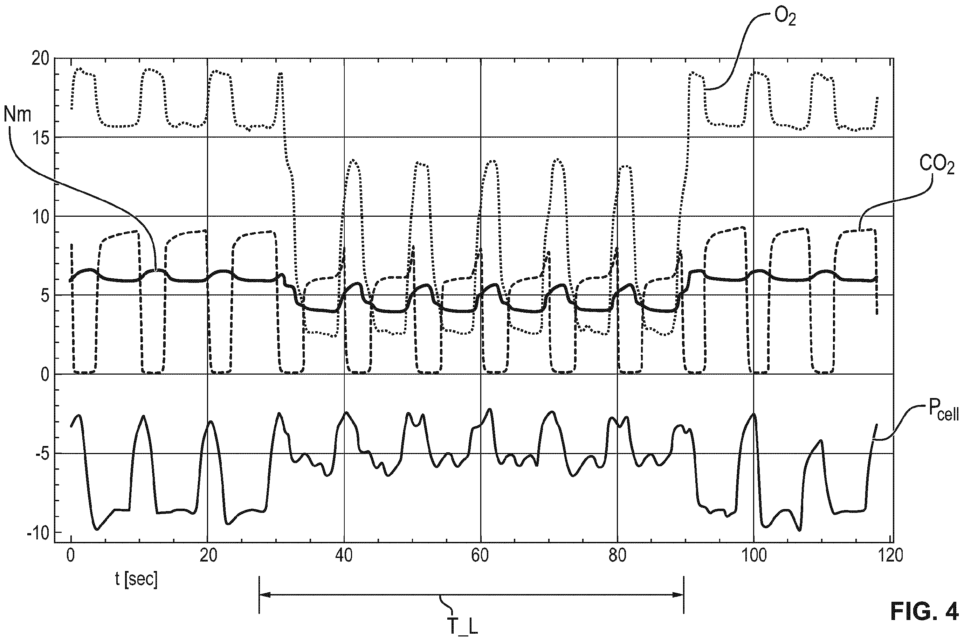

Figur 4 zeigt

- den zeitlichen Verlauf der CO2-Konzentration,
- den zeitlichen Verlauf der O2-Konzentration,
- den zeitlichen Verlauf der Konzentration eines Narkosemittels (mit Nm bezeichnet) und
- den zeitlichen Verlauf des Drucks $P_{cell}$ in einem Messpunkt der Gassensor-Anordnung 50.

In der Zeitspanne von 0 bis 120, welche in Figur 4 gezeigt ist, ist die Pumpe 55 kontinuierlich eingeschaltet und saugt Gas an. Die vier zeitlichen Verläufe CO2, O2, Nm und $P_{cell}$ wurden so skaliert, dass sie auf derselben Skala dargestellt werden können. Auf der x-Achse ist die Zeit in sec aufgetragen.

Zu sehen ist einerseits, dass die Schwankungen der Konzentrationen von CO2, O2 und Nm den Schwankungen des Drucks $P_{cell}$ hinterhereilen, weil der Druck $P_{cell}$ sich annähernd mit Schallgeschwindigkeit fortpflanzt und eine Fortpflanzung der veränderlichen Gaskonzentrationen in dem Entnahmeschlauch 52 deutlich länger als der Druck benötigt, um die Gassensor-Anordnung 50 zu erreichen, beispielsweise bei einem 3 m langen Entnahmeschlauch 52 etwa 2 sec.

Im Zeitraum T_L ist eine Leckage L aufgetreten. Diese Leckage L führt zu einer deutlich geringeren O2-Konzentration und zu einer etwas geringeren CO2-Konzentration im Zeitraum T_L. Außerdem verändern die Spitzen des CO2-Verlaufs und des O2-Verlaufs ihre Formen. Der CO2-Verlauf zeigt im Zeitraum T_L pro Atemzug jeweils eine "Nase" (peak), welche zeitlich nahe dem Ende der Exspirationsphase auftritt. Der O2-Verlauf hat eine eher trapezförmige als rechteckige Form.

Die Signalverarbeitungseinheit 30 ermittelt den jeweiligen zeitlichen Verlauf der O2-Konzentration, der CO2-Konzentration und optional auch den der N2O-Konzentration und den der Narkosemittel-Konzentration.

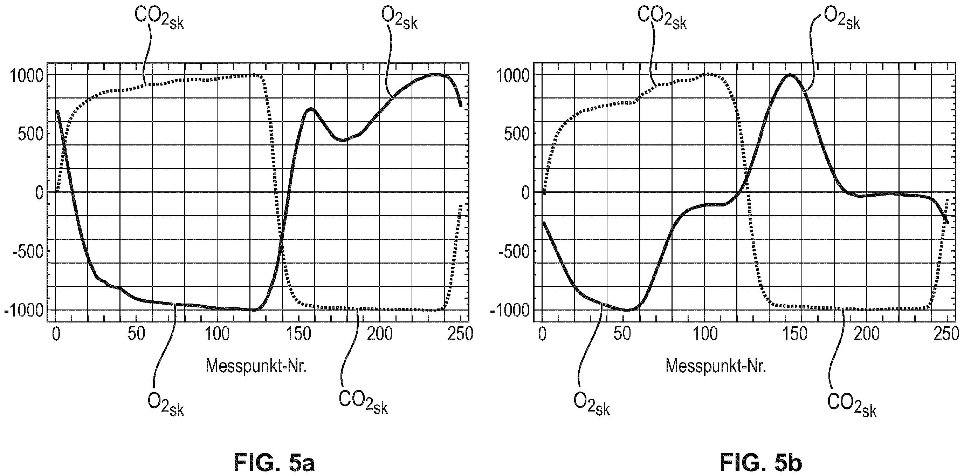

Die Signalverarbeitungseinheit 30 ermittelt aufgrund des CO2-Verlaufs und / oder des O2-Verlaufs jeden Atmungsvorgang und die Exspirationsphase dieses Atmungsvorgangs während der künstlichen Beatmung des Patienten 20. In einer Ausgestaltung ist jede Exspirationsphase eine solche Zeitspanne zwischen zwei Nulldurchgängen der normierten CO2-Konzentration, in denen die normierte CO2-Konzentration größer als Null ist, vgl. Figur 5. Zu jedem Abtast-Zeitpunkt t(i) während eines Exspirationsvorgangs liegt jeweils ein Messwert CO2(i) und O2(i) für die CO2-Konzentration und für die O2-Konzentration am Zeitpunkt t(i) vor. Auf der x-Achse ist wiederum die jeweilige Nummer des Messpunkts aufgetragen. Die Abtastfrequenz beträgt im gezeigten Beispiel 50 Hz.

Die Signalverarbeitungseinheit 30 normiert die Messwerte, die während eines Exspirationsvorgangs gemessen worden sind, auf einen Bereich zwischen -A und +A, wobei A ein vorgegebener Wert ist. Für die Messwerte der O2-Konzentration wird diese Normierung bevorzugt gemäß der Rechenvorschrift

$$O2_{sk}(i) = A * \frac{2*O2(i) - [O2_{max} + O2_{min}]}{O2_{max} - O2_{min}}$$

durchgeführt, für die Messwerte der CO2-Konzentration durch eine entsprechende Rechenvorschrift. Durch diese Normierung wird auch ein konstanter Offset rechnerisch beseitigt. In dieser Rechenvorschrift bezeichnen $O2_{max}$ und $O2_{min}$ den größten bzw. den kleinsten Wert für die O2-Konzentration während eines Exspirationsvorgangs. Figur 5 a) zeigt den normierten Verlauf des Beispiels von Figur 2. Figur 5 b) zeigt den normierten Verlauf des Beispiels von Figur 3. In diesem Falle beträgt A = 1000. Selbstverständlich sind auch ein anderer Wert für A und eine andere Rechenvorschrift für die Normierung möglich.

Die Signalverarbeitungseinheit 30 berechnet zeitliche Veränderungen in dem O2-Verlauf und in dem CO2-Verlauf, beispielsweise gemäß der Rechenvorschrift

$$\Delta O2_{sk}(i) = O2_{sk}(i) - O2_{sk}(i\text{-}s_{O2}) \text{ sowie}$$

$$\Delta CO2_{sk}(i) = CO2_{sk}(i) - CO2_{sk}(i\text{-}s_{CO2}),$$

wobei $s_{O2}$ und $s_{CO2} >= 1$ zwei vorgegebene oder auch berechnete Anzahlen (Schrittweiten) sind. Dadurch werden zwei Differenzen-Verläufe $\Delta O2_{sk}$ und $\Delta CO2_{sk}$ berechnet. Bevorzugt liegen die Schrittweiten $s_{O2}$ und $s_{CO2}$ zwischen 5 und 50. Die Abtast-Frequenz beträgt beispielsweise 50 Hz. Zwischen den beiden Abtast-Zeitpunkten i und i-$s_{CO2}$ liegt bevorzugt eine Zeitspanne zwischen 5 * 20 msec = 100 msec und 50 * 20 msec = 1 Sekunde. Diese zeitlichen Veränderungen $\Delta O2_{sk}$ und $\Delta CO2_{sk}$ korrelieren mit den Ableitungen der Konzentrations-Verläufe nach der Zeit.

Bevorzugt wird die im Folgenden beschriebene Synchronisierung der beiden zeitlichen Verläufe durchgeführt. Möglich ist, für O2 und für CO2 unterschiedliche Schrittweiten $s_{O2}$ und $s_{CO2}$ zu verwenden.

Die Schrittweiten $s_{O2}$ und $s_{CO2}$ haben einen Einfluss darauf, wie zuverlässig eine Leckage L erkannt wird und ausreichend sicher von einem leckfreien Zustand unterschieden werden kann. In einer Ausgestaltung werden probeweise unterschiedliche mögliche Schrittweiten $s_{O2}$ bzw. $s_{CO2}$ für den abgetasteten Verlauf der O2-Konzentration und für den der CO2-Konzentration erprobt. Welche Schrittweiten $s_{O2}$ und $s_{CO2}$ zu guten Detektionsergebnissen führen, kann von der Beatmungsfrequenz abhängen. Möglich ist, vorab für unterschiedliche Werte von Parametern der künstlichen Beatmung jeweils einen Satz von gut geeigneten Schrittweiten $s_{O2}$ und $s_{CO2}$ zu ermitteln und abzuspeichern. Zu den Parametern gehören insbesondere die Frequenz und die Amplituden der Beatmungshübe sowie das zeitliche Verhältnis von Inspiration zu Exspiration. Wenn das Verfahren durchgeführt werden soll, werden die bei der künstlichen Beatmung aktuell verwendeten Werte der Parameter ermittelt, und der zugeordnete abgespeicherte Satz von Schrittweiten $s_{O2}$ und $s_{CO2}$ wird verwendet.

Falls die beiden ermittelten und tatsächlich verwendeten Schrittweiten sich voneinander unterscheiden, wird eine Phasenverschiebung bewirkt, die bevorzugt rechnerisch ausgeglichen wird. Zum rechnerischen Ausgleichen werden beispielsweise die beiden Differenzen-Verläufe $\Delta O2_{sk}$ und $\Delta CO2_{sk}$ relativ zueinander zeitlich verschoben, bis das Minimum des Verlaufs $\Delta CO2_{sk}$ und das Maximum des Verlaufs $\Delta O2_{sk}$ nach der Verschiebung auf demselben Zeitpunkt liegen.

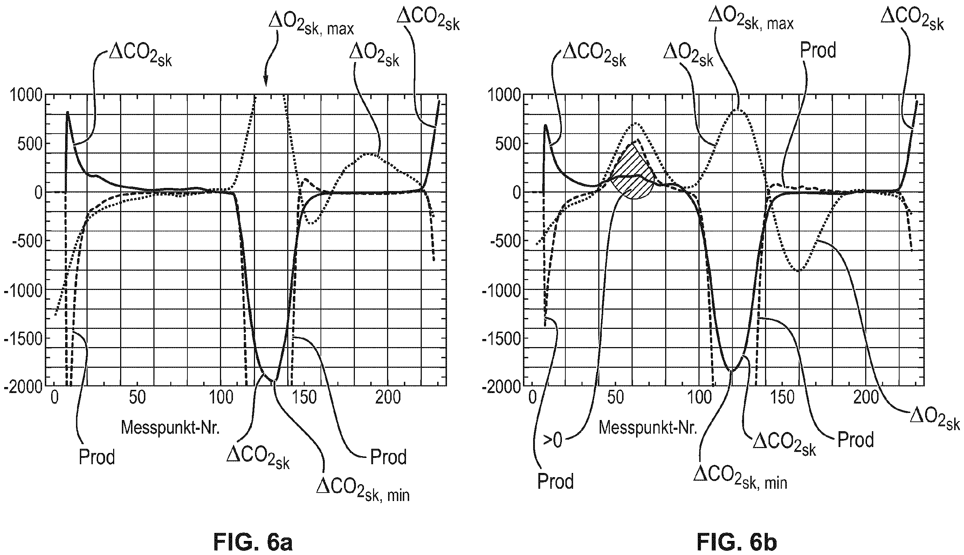

Eine solche Schrittweite $s_{O2}$ für O2 und eine solche Schrittweite $s_{CO2}$ für CO2 werden ermittelt und für das folgende Verfahren verwendet, bei dem das Maximum von $\Delta O2_{sk}$, also das Maximum der Veränderungen der normierten O2-Werte, und das Minimum von $\Delta CO2_{sk}$, also das Minimum der Veränderungen der normierten CO2-Werte, in demselben Zeitpunkt liegen, optional nach der gerade beschriebenen zeitlichen Verschiebung. Das Maximum ist in Figur 6 mit $\Delta O2_{sk,max}$ bezeichnet, das Minimum mit $\Delta CO2_{sk,min}$. Anschließend berechnet die Signalverarbeitungseinheit 30 für eine Abfolge von Abtast-Zeitpunkten während eines Exspirationsvorgangs eine Abfolge von arithmetischen Produktwerten gemäß der Rechenvorschrift

$$Prod(i) = \Delta O2_{sk}(i) * \Delta CO2_{sk}(i).$$

Falls keine Leckage L auftritt, so gilt in der Regel zu jedem Abtastzeitpunkt t(i) während eines Exspirationsvorgangs $\Delta CO2_{sk}(i) > 0$ (CO2-Konzentration steigt an) und $\Delta O2_{sk}(i) < 0$ (O2-Konzentration fällt ab). Während eines Inspirationsvorgangs gilt in der Regel umgekehrt $\Delta CO2_{sk}(i) < 0$ und $\Delta O2_{sk}(i) > 0$. Daher gilt in der Regel während eines gesamten Atemzugs Prod(i) < 0, weil $\Delta CO2_{sk}(i)$ und $\Delta O2_{sk}(i)$ verschiedene Vorzeichen aufweisen, vorausgesetzt keine Leckage L ist aufgetreten. Falls hingegen eine Leckage L aufgetreten ist, so gilt für einzelne Abtast-Zeitpunkte t(i) Prod(i) > 0, d.h. gleiches Vorzeichen.

Figur 6 a) zeigt die nach der gerade beschriebenen Synchronisation resultierenden zeitlichen Verläufe von $\Delta CO2_{sk}$, $\Delta O2_{sk}$ und Prod, die aus dem Beispiel von Figur 2 resultieren (keine Leckage L aufgetreten). Figur 6b) zeigt die zeitlichen Verläufe von $\Delta CO2_{sk}$, $\Delta O2_{sk}$ und Prod, die aus dem Beispiel von Figur 3 resultieren (Leckage L aufgetreten). In Figur 6 b) wird weiterhin ein mit >0 gekennzeichneter Bereich dargestellt, in dem Prod(i) > 0 gilt.

Die Signalverarbeitungseinheit 30 berechnet bevorzugt die Produktwerte Prod(i) nur für jeden Exspirationsvorgang, aber nicht für einen Inspirationsvorgang. Eine Leckage L wirkt sich während eines Exspirationsvorgangs deutlich stärker aus als während eines Inspirationsvorgangs, was in Figur 3 gut zu sehen ist. Eine Leckage L wird auch in dieser Ausgestaltung rasch genug detektiert, und Rechenzeit wird eingespart. Im Beispiel von Figur 6 findet in der Zeitspanne zwischen dem 1. und dem 120. Messpunkt ein Exspirationsvorgang statt.

Eine Leckage L soll von sonstigen Störeinflüsse unterschieden werden können. Ein sonstiger Störeinfluss kann beispielsweise daraus resultieren, dass Flüssigkeitstropfen oder Kondensat die Membrane der Wasserfalle 51 so lange verstopfen, bis kondensierte Flüssigkeit in einen Tank der Wasserfalle 51 tropft oder in diesen transportiert wird und dann die Membrane schlagartig eine höhere Durchlässigkeit aufweist. Diese schlagartig höhere Durchlässigkeit könnte eine in Wirklichkeit nicht vorhandene Leckage vortäuschen.

In einer Ausgestaltung berechnet die Signalverarbeitungseinheit 30 die Summe DiPhaC über alle Produktwerte Prod(i) während eines Exspirationsvorgangs, die größer als 0 sind. DiPhaC bedeutet "Differential in Phase Covariance". Bis auf numerische Abweichungen ist diese Summe DiPhaC proportional zu der Fläche des in Figur 6 b) mit >0 gekennzeichneten Bereichs. Falls diese Summe DiPhaC oberhalb einer vorgegebenen Schranke liegt, so hat die Signalverarbeitungseinheit 30 eine Leckage L detektiert oder wenigstens ein Indiz dafür, dass eine Leckage L aufgetreten ist. In einer Ausgestaltung generiert die Signalverarbeitungseinheit 30 daraufhin eine Nachricht, dass eine Leckage L detektiert worden ist.

Das gerade beschriebene Verfahren mit der Summe DiPhaC hat mehrere Vorteile im Vergleich zu anderen Verfahren, um eine Leckage L zu erkennen. Solange eine Leckage L auftritt, ist Prod(i) > 0. Sobald die Leckage L gestopft oder anderweitig beseitigt ist, gilt wieder Prod(i) <= 0. Das Verfahren mit DiPhaC vermag daher nicht nur eine schlagartig auftretende Leckage L zu detektieren, sondern auch eine sich allmählich vergrößernde Leckage L, das beispielsweise aus einer Materialermüdung resultieren kann. Die vorteilhafte Wirkung, dass verschiedene Arten von Leckagen detektiert werden können, resultiert insbesondere daraus, dass das Verfahren mit DiPhaC nicht erfordert, einen aktuellen Messwert oder einen hergeleiteten Wert mit einem Referenzwert, der sich auf einen früheren Abtast-Zeitpunkt bezieht, zu vergleichen.

Das Verfahren mit DiPhaC hat außerdem folgenden Vorteil im Vergleich zu dem möglichen Vorgehen, ein statistisches Maß für die Phasenverschiebung zwischen der CO2-Konzentration und der O2-Konzentration zu berechnen und dieses statistische Maß mit einer Schranke zu vergleichen: Die Schranke, mit der das statistische Maß verglichen wird, muss an mindestens einen Parameter der Beatmung angepasst werden, beispielsweise an den Druck oder die Frequenz der künstlichen Beatmung oder an einen physiologischen Zustand des Patienten 20. Häufig wird diese Schranke abhängig von Messwerten automatisch festgelegt, wobei diese Messwerte während eines leckfreien Zustands gemessen werden. Das Verfahren mit DiPhaC erfordert kein solches Vorwissen oder Hintergrundwissen, um eine Schranke festzulegen, und auch keinen leckfreien Zustand. Daher wird keine Initialisierungsphase benötigt.

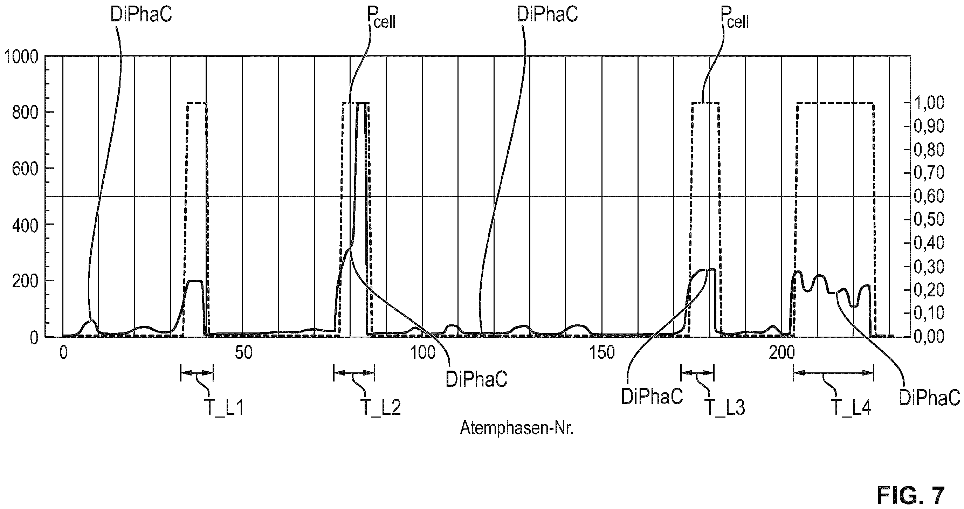

Figur 7 zeigt das Ergebnis eines Versuchs, bei dem gezielt eine Leckage erzeugt wurde. Um eine Leckage L gezielt zu erzeugen, wurde in einem Bereich am oder nahe des Eingangs der Gassensor-Anordnung 50, nämlich an der Stelle L von Figur 1, ein ansteuerbares Magnetventil mit einem Rubin-Lochstein eingebaut. Bei voll geöffneter Stellung des Magnetventils wird eine Leckage L hergestellt, das so groß ist, dass der Volumenfluss der durch die Leckage L eingesaugten Umgebungsluft etwa 20 % des gesamten Volumenflusses des Gasgemischs (Atemgasprobe und Umgebungsluft) beträgt, welches durch die Gassensor-Anordnung 50 fließt. Falls eine solche Leckage L nicht beseitigt wird, werden die Messergebnisse der Gassensor-Anordnung 50 erheblich verfälscht.

In den Zeiträumen T_L1, ..., T_L4 wurde jeweils eine Leckage L erzeugt. Auf der rechten y-Achse ist eine Skala für die Stellung des Ventils von 0 (vollständig geschlossen) bis 1 (vollständig geöffnet) dargestellt. Auf der linken y-Achse ist eine Skala für DiPhaC dargestellt. Das Diagramm von Figur 7 zeigt den zeitlichen Verlauf des Drucks $P_{cell}$ an der Gassensor-Anordnung 50 und den zeitlichen Verlauf von DiPhaC. Der Druck $P_{cell}$ wurde über jeweils eine Atemphase des Patienten 20 gemittelt, sodass jeder Messwert sich auf genau eine Atemphase bezieht. Auf der x-Achse sind die fortlaufenden Nummern der Atemphasen eingetragen. Die Dauer eine Atemphase kann zeitlich variieren. Für CO2 wurden als beispielhafte Schrittweiten $s_{CO2}$ = 26 und $s_{O2}$ = 25 verwendet. Zu sehen ist, dass eine Leckage L stets zu einem Wert für DiPhaC größer als 1000 führt, während sonstige Effekte stets zu einem kleineren Wert führen, nämlich weniger als die Hälfte. Daher wird in diesem Fall jedes Auftreten einer Leckage L erkannt, und ein Fehlalarm wird vermieden.

Bevorzugt wird das Verfahren, das auf dem Signal DiPhaC beruht, um ein weiteres Verfahren ergänzt. Somit werden zwei Leckage-Kriterien verwendet, nämlich DiPhaC als erstes Leckage-Kriterium und ein zweites Leckage-Kriterium mit der Bezeichnung DPC ("Delta Pressure Covariance"). Dieses weitere Verfahren vermag mit größerer Sicherheit eine schlagartig auftretende Leckage sicher zu detektieren, und zwar auch bei einem niedrigen oder nur relativ wenig variierenden Beatmungsdruck und / oder bei einer hohen Beatmungsfrequenz. Ein niedriger Beatmungsdruck oder eine geringe Amplitude des Beatmungsdrucks in Verbindung mit einer hohen Beatmungsfrequenz werden insbesondere dann angewendet, wenn ein Kind künstlich beatmet wird.

Die Signalverarbeitungseinheit 30 berechnet während jeder Exspirationsphase jeweils mehrmals Werte für die Covarianz Cov[CO2,O2] zwischen dem zeitlichen Verlauf der CO2-Konzentration und dem zeitlichen Verlauf der O2-Konzentration. Diese Covarianz Cov[CO2,O2] variiert mit der Zeit. Für mehrere aufeinanderfolgende Abtast-Zeitpunkte berechnet die Signalverarbeitungseinheit 30 einen Wert für die Covarianz Cov[CO2,O2] unter Verwendung von jeweils n Werten für die CO2-Konzentration und die CO2-Konzentration.

Die Signalverarbeitungseinheit 30 wendet beispielsweise die folgende Rechenvorschrift an:

$$Cov[CO2,O2](m) = \frac{2}{n} \sum_{i=1}^{n} \frac{CO2(m+i) - CO2_{avg}}{CO2_{max} - CO2_{min}} * \frac{O2(m+i) - O2_{avg}}{O2_{max} - O2_{min}}$$

In dieser Rechenvorschrift bezeichnen $O2_{max}$ und $O2_{min}$ den größten bzw. den kleinsten Wert für die O2-Konzentration unter den n Werten für die n Abtast-Zeitpunkte t(m+1), ..., t(m+n). $O2_{avg}$ bezeichnet den arithmetischen Mittelwert dieser n Werte. Diese Covarianz Cov[CO2,O2] ist ein Maß für die Phasenverschiebung zwischen der zeitlich veränderlichen CO2-Konzentration und der zeitlich veränderlichen O2-Konzentration in dem Messzeitraum. Falls keine Leckage aufgetreten ist, so beträgt die Covarianz idealerweise -1, in der Praxis liegt sie zwischen -0,6 und -0,8.

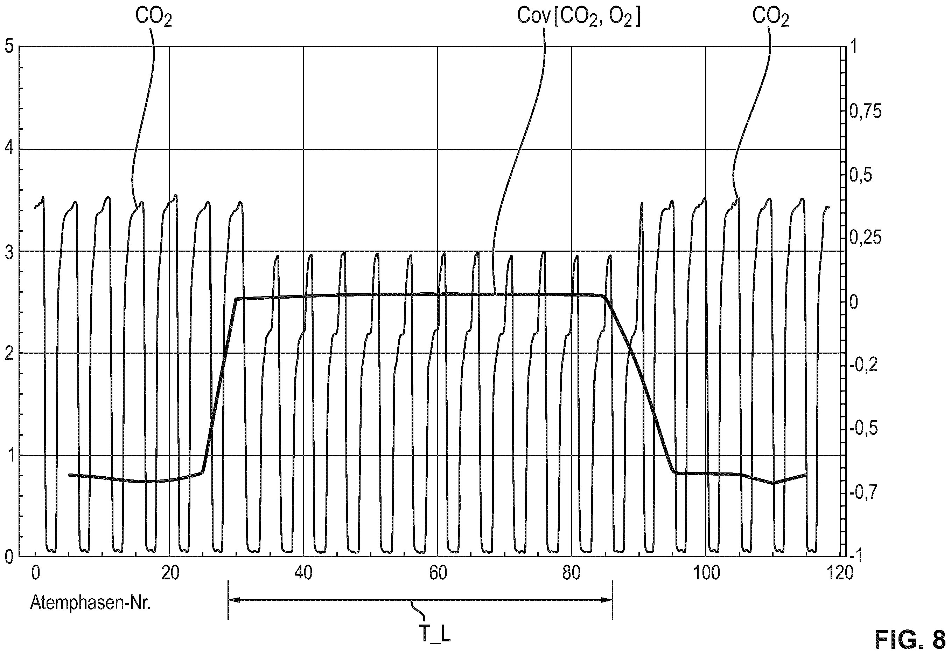

Figur 8 zeigt beispielhaft den zeitlichen Verlauf der CO2-Konzentration und den zeitlichen Verlauf der Covarianz Cov[CO2,O2] zwischen CO2 und O2. Auf der linken y-Achse ist eine Skala für die CO2-Konzentration aufgetragen, auf der rechten y-Achse eine Skala für die Covarianz. Auf der x-Achse ist wiederum die laufende Nummer der Atemphasen aufgetragen.

Die Covarianz Cov[CO2,O2] - oder ein sonstiges Maß für die Phasenverschiebung zwischen den beiden zeitlichen Verläufen CO2 und O2 - ist in der Zeitspanne, in der eine Leckage auftritt, größer als in einer leckfreien Zeitspanne. Jedoch wird der Wert der Covarianz Cov[CO2,O2] nicht nur vom Auftreten und der Größe einer Leckage L beeinflusst, sondern von einer Vielzahl weiterer Faktoren, insbesondere vom Beatmungsdruck, der Beatmungsfrequenz und ob die Beatmung volumengesteuert oder druckgesteuert durchgeführt wird.

Das nachfolgend beschriebene Vorgehen vermag in vielen Fällen diesen Nachteil zu beseitigen, ohne dass die Auswirkungen der weiteren Faktoren auf die Covarianz Cov[CO2,O2] bekannt sein müssen. Ein zugrundeliegendes Konzept ist das Vorgehen, die zeitliche Änderung der Covarianz zu berechnen und auszuwerten. Diese Änderung der Covarianz Cov[CO2,O2] zeigt eine plötzlich auftretende Leckage L an.

Die Signalverarbeitungseinheit 30 berechnet weiterhin eine Änderung

$$\Delta Cov[CO2,O2](m) = Cov[CO2,O2](m) - Cov[CO2,O2](m-s_{Cov})$$

der Covarianz Cov[CO2,O2] mit einer vorgegebenen Schrittweite scov, beispielsweise scov=1. Weiterhin berechnet die Signalverarbeitungseinheit 30 eine Änderung

$$\Delta P_{Cell}(j) = P_{Cell}(j) - P_{Cell}(j-s_{Cell})$$

des Drucks $P_{Cell}$. Im gezeigten Beispiel ist jeder verwendete Wert für diesen Druck $P_{Cell}$ über jeweils eine Atemphase gemittelt. Der Abtastzeitpunkt t(j) für den Druck $P_{Cell}$ wird geeignet relativ zu der Covarianz Cov[CO2,O2] zeitlich positioniert, beispielsweise j = m+n. Diese zeitliche Positionierung kann

- von dem Abstand zwischen der Gassensor-Anordnung 50, welche die Konzentrationen misst, und einem Messpunkt, an dem der Drucksensor 57 den Druck misst, sowie
- von der Fließgeschwindigkeit der abgesaugten Atemgasprobe durch den Entnahmeschlauch 52 hindurch abhängen.

Der Druck $P_{cell}$ kann sich beispielsweise dann rasch ändern, wenn eine größere Menge von Fluid, insbesondere auskondensiertes Wasser, mit dem Strom der abgesaugten Atemgasprobe in das Mess-System 110 gelangt und sich vor der Membrane der Wasserfalle 51 sammelt. Wenn diese Fluidmenge dann schlagartig in den Tank der Wasserfalle 51 fließt oder gefördert wird, so wird die Membrane schlagartig freigegeben.

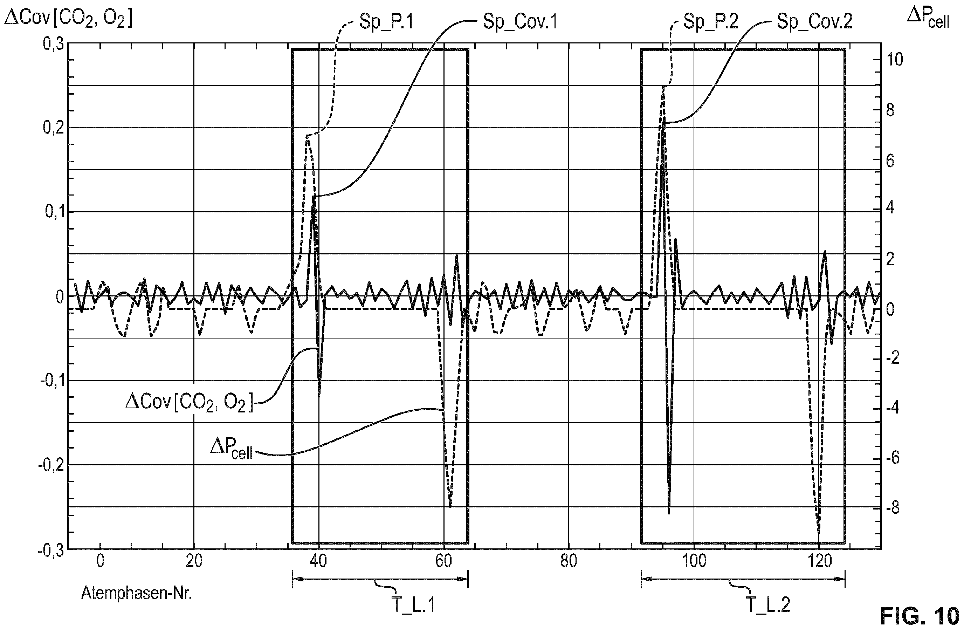

Figur 10 zeigt beispielhaft die zeitlichen Verläufe von $\Delta Cov[CO2,O2]$ und $\Delta P_{Cell}$. Auf der x-Achse sind wiederum die laufenden Nummern der Atemphasen des Patienten 20 aufgetragen. Auf der linken y-Achse ist die Skala für die dimensionslose Größe $\Delta Cov[CO2,O2]$ aufgetragen, auf der rechten y-Achse die Skala für die Größe $\Delta P_{Cell}$ in hPa. In den beiden Zeiträumen T_L.1 und T_L.2 ist eine Leckage L aufgetreten. Das erste Auftreten der Leckage L führt zu der Spitze Sp_P.1 des Verlaufs $\Delta P_{Cell}$ und der Spitze Sp_Cov.1 des Verlaufs $\Delta Cov$, das zweite Auftreten zu den Spitzen Sp_P.1 und Sp_Cov.1, also zu schlagartig ansteigenden Werten. Das Verschließen der Leckage L führt zu jeweils zwei Spitzen in der entgegengesetzten Richtung, also zu schlagartig abfallenden Werten.

Die Signalverarbeitungseinheit detektiert eine Leckage L abhängig von den Werten für $\Delta Cov[CO2,O2]$ und $\Delta P_{Cell}$. Falls beispielsweise zu einem Abtastzeitpunkt t(j) sowohl $\Delta Cov[CO2,O2](j)$ als auch $\Delta P_{Cell}(j)$ jeweils oberhalb einer vorgegebenen Schranke liegen, so ist zu diesem Abtastzeitpunkt t(j) eine Leckage L aufgetreten. Dieses Verfahren liefert ein Signal, das im Folgenden als "Delta Pressure Covariance" (DPC) bezeichnet wird. Möglich ist auch, die Covarianz zwischen $\Delta Cov[CO2,O2]$ und $\Delta P_{Cell}$ zu berechnen. Falls diese Covarianz oberhalb einer vorgegebenen Schranke liegt, beispielsweise größer 0 wird, so ist eine Leckage L detektiert.

Das Verfahren mit dem Signal DPC erkennt häufig auch bei geringen Beatmungsdrücken, also geringen Amplituden des CO2-Verlaufs und des O2-Verlaufs, sowie bei hohen Atemfrequenzen zuverlässig eine plötzlich auftretende Leckage L. Es erzeugt nur relativ wenige Fehlalarme, oft überhaupt keine Fehlalarme.

Bevorzugt werden beide Verfahren DiPhaC und DPC verwendet, um eine Leckage zu detektieren. Eine Leckage L ist detektiert, wenn entweder DiPhaC oder DPC oder beide Kriterien das Auftreten einer Leckage L signalisieren. Möglich ist aber auch, nur eines der beiden Kriterien anzuwenden.

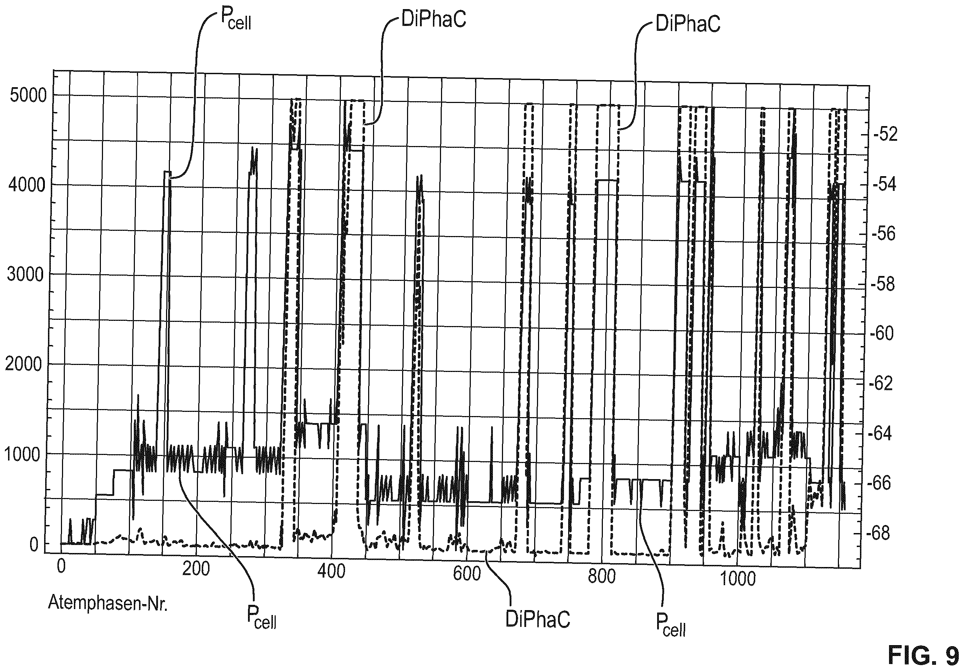

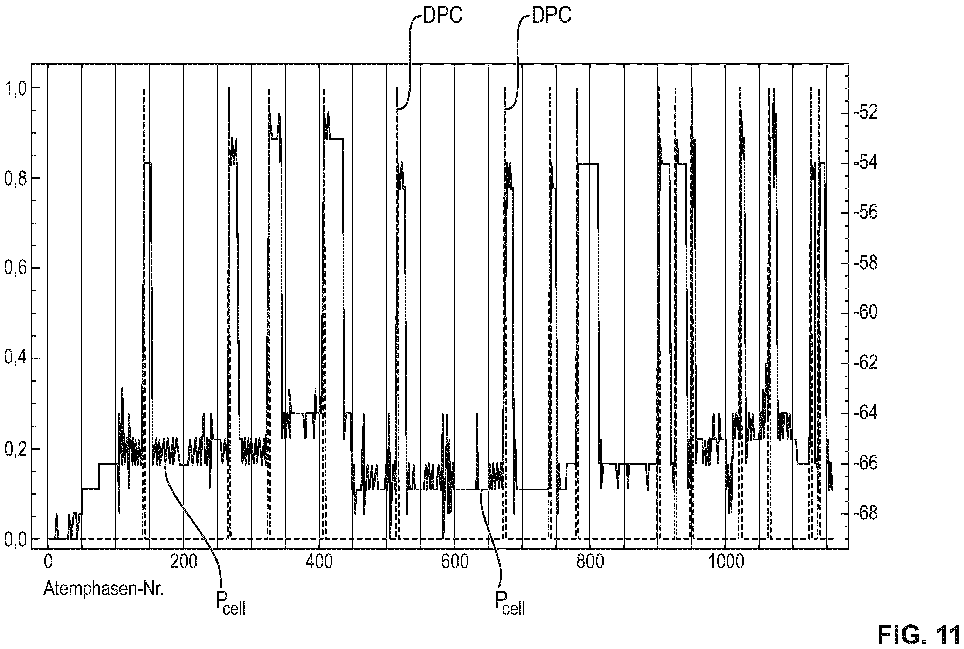

Figur 9 und Figur 11 zeigen die beiden Verfahren DiPhaC und DPC zur Erkennung einer Leckage L, wobei ein Patient 20 mit relativ hoher Frequenz und relativ geringer Amplitude des Beatmungsdrucks künstlich beatmet wird und wobei durch Versuche mithilfe des oben beschriebenen ansteuerbaren Magnetventils nacheinander 15 mal jeweils eine plötzlich auftretende Leckage erzeugt wurde. Figur 9 und Figur 11 zeigen jeweils den Verlauf des Drucks $P_{Cell}$ am Eingang der Gassensor-Anordnung 50, wobei wiederum jeder dargestellte Messwert den über eine Atemphase des Patienten gemittelten Druck $P_{Cell}$ angibt und auf der x-Achse die laufenden Nummern der Atemphasen des Patienten 20 aufgetragen sind. Pro Atemphase wird jeweils ein Wert des Drucks $P_{cell}$ dargestellt. Dieser dargestellte Wert wird beispielsweise durch Mittelung über mehrere Messwerte erzeugt. Die rechte y-Achse zeigt in beiden Figuren die Werte von -68 bis -52 des Drucks $P_{Cell}$ in hPa an. Figur 9 zeigt weiterhin den zeitlichen Verlauf des Signals DiPhaC, Figur 11 den des Signals DPC. Die linke y-Achse zeigt in beiden Figuren eine Skala für dieses Signal. Das Signal DPC nimmt nur die Werte 0 und 1 an. In dieser Situation vermag das Verfahren DiPhaC die erste und die zweite plötzlich auftretende Leckage L nicht zu detektieren, während das Verfahren DPC alle 15 auftretenden Leckagen L automatisch detektiert.

Wenn durch mindestens eines der Verfahren DiPhaC oder DPC eine Leckage L erkannt worden ist, so übermittelt die Gassensor-Anordnung 50 eine entsprechende Nachricht an das Steuergerät 35. In einer Ausgestaltung löst das Steuergerät 35 nach Erhalt einer solchen Nachricht unverzüglich einen Alarm in einer von einem Menschen wahrnehmbaren Form aus.

In einer anderen Ausgestaltung löst das Steuergerät 35 nach Erhalt einer solchen Nachricht zunächst eine Überprüfung aus, ob tatsächlich eine Leckage L aufgetreten ist, welches unverzüglich beseitigt werden muss, oder aber ein sonstiges Ereignis, welches eine Leckage L vortäuscht, aber kein Eingreifen erforderlich macht. Diese Überprüfung reduziert die Anzahl von Fehlalarmen, ohne dass eine tatsächlich auftretende Leckage L übersehen wird. Die künstliche Beatmung des Patienten 20 wird während diese Überprüfung fortgesetzt und ist durch die Überprüfung nicht beeinträchtigt. Im Ausführungsbeispiel wird diese Überprüfung durchgeführt, wenn das Beatmungsgerät 100 einen positiven Beatmungsdruck oberhalb einer vorgegebenen Schranke erzielt, also Atemgas ausstößt.

Die Überprüfung, ob tatsächlich eine Leckage L aufgetreten ist, umfasst in einer Ausgestaltung die folgenden Schritte:

- Die Pumpe 55 der Gassensor-Anordnung 50 wird für die Dauer der Überprüfung abgeschaltet.
- Der Drucksensor 58 misst weiterhin den Umgebungsdruck $P_{amb}$.
- Der Drucksensor 57 der Gassensor-Anordnung 50 misst weiterhin den Druck $P_{cell,abs}$, der am Ende des Entnahmeschlauchs 52 anliegt.
- Der Drucksensor 36 im Beatmungskreislauf 40 misst weiterhin den Druck im Beatmungskreislauf 40, beispielsweise den Druck $P_{aw}$.

Der Druck pflanzt sich bekanntlich mit Schallgeschwindigkeit fort. Bis auf unvermeidliche Messfehler und dem Prozessrauschen stimmt daher der zeitliche Verlauf des Drucks $P_{cell}$ im Entnahmeschlauch 52 mit dem zeitlichen Verlauf des Drucks $P_{aw}$ im Beatmungskreislauf 40 überein, vorausgesetzt es ist keine Leckage aufgetreten. Weil die Pumpe 55 abgeschaltet ist, hat eine mögliche Verstopfung oder sonstige Beeinträchtigung der Membrane an der Wasserfalle 51 keinen Einfluss auf die Messung. Nicht benötigt wird ein Referenzwert, der vor Überprüfung auf die Leckage gemessen worden ist.

In einer Ausgestaltung wird die Entscheidung, ob tatsächlich eine Leckage L aufgetreten ist oder nicht, wie folgt getroffen: Die Pumpe 55 wird abgeschaltet. Anschließend werden die Drücke $P_{cell}$ im Entnahmeschlauch 52 und Paw im Beatmungskreislauf 40 miteinander verglichen. Für diesen Vergleich werden an n aufeinanderfolgenden Abtast-Zeitpunkten t(1), ..., t(n)

- der Druck im Beatmungskreislauf 40, beispielsweise der am Patienten 20 anliegende Beatmungsdruck Paw,
- der Druck $P_{cell,abs}$, der an der Gassensor-Anordnung 50 anliegt, sowie
- der Umgebungsdruck $P_{amb}$

gemessen. Der Umgebungsdruck $P_{amb}$ wird von den Drucksensor 58 gemessen und insbesondere deshalb gemessen und verwendet, weil er zeitlich variieren kann und diese zeitliche Variation nicht den Vergleich von relativen Drücken verfälschen soll.

Die Größe

$$\Delta P_{max} = Max\{P_{aw}[t(1)], \ldots, P_{aw}[t(n)]\} -$$

$$Max\{P_{cel,absl}[t(1)]-P_{amb}[t(1)], \ldots, P_{cell,abs}[t(n)]-P_{amb}[t(n)]\}$$

wird berechnet. Idealerweise ist diese Größe $\Delta P_{max}$ gleich dem maximalen Druckverlust, der zwischen dem Beatmungskreislauf 40 und der Gassensor-Anordnung 50 auftritt und sich bei Auftreten einer Leckage L verändert. Abhängig von dem Beatmungsdruck $P_{Insp}$, den die Förderereinheit 24a im Beatmungskreislauf 40 während des Inspirationsvorgangs erzeugt, sowie abhängig von der Beatmungsrate RR wird eine Druckschranke $P_{limit}$ berechnet. Eine Leckage L ist detektiert, wenn

$\Delta P_{max} > P_{limit}$

gilt. Falls keine Leckage aufgetreten ist, so hat idealerweise der Umgebungsdruck $P_{amb}$ keinen Einfluss auf die berechnete Größe, und es gilt $\Delta P_{max} = 0$.

Das gerade beschriebene Vorgehen berücksichtigt den Umgebungsdruck $P_{amb}$, der sich nur bei einer Leckage L signifikant auf die berechnete Größe $\Delta P_{max}$ auswirkt, und vermag daher besser als andere Verfahren eine Leckage L von einer Störgröße zu unterscheiden.

**Bezugszeichenliste**

| | |
|---|---|
| 20 | Patient, der künstlich beatmet wird , mit der patientenseitigen Koppeleinheit 21 verbunden |
| 21 | patientenseitige Koppeleinheit in Form eines Mundstücks oder einer Atemmaske, mit dem Y-Stück 22 verbunden |
| 22 | Y-Stück, welche die patientenseitige Koppeleinheit 21 mit einer Zufuhrleitung für die Zufuhr von Gas (Einatmung, Inspiration) und eine Abfuhrleitung für die Abfuhr von Gas (Ausatmung, Exspiration) verbindet |
| 23a | Rückschlagventil, das in der Einatmungs-Leitung 32 einen Gasfluss in Richtung des Patienten 20 durchlässt und in Gegenrichtung gesperrt |
| 23b | Rückschlagventil, das in der Ausatmungs-Leitung 33 einen Gasfluss weg vom Patienten 20 durchlässt und in Richtung zum Patienten 20 hin sperrt |
| 24a | Förderereinheit, welche einen Volumenstrom in Richtung des Patienten 20 erzeugt |
| 24b | PEEP-Ventil, welches einen Druck in der Lunge des Patienten 20 aufrechterhält |
| 25a | Kohlendioxidabsorber, absorbiert aus dem Beatmungskreislauf 40 Kohlendioxid |
| 26 | Handbeatmungsbeutel, über den der Beatmungskreislauf 40 angetrieben werden kann |
| 27 | Fluidstrom von Frischluft oder sonstigem Frischgas zum Beatmungskreislauf 40 |
| 28 | Fluidstrom von dampfförmigem Narkosemittel zum Beatmungskreislauf 40 |
| 29 | einstellbares Überdruckventil, welches Gas aus dem Beatmungskreislauf 40 abzulassen vermag |
| 30 | datenverarbeitende Signalverarbeitungseinheit für die Gassensor-Anordnung 50, wertet Signale von den Sensoren 53, 54, 57 und 58 aus, vermag eine Leckage L zu detektieren und eine Nachricht an das Steuergerät 35 zu übermitteln |
| 31 | Narkosemittelverdampfer, erzeugt den Narkosemittelstrom 28 |
| 32 | Atemgasleitung für die Einatmung, mit dem Y-Stück 22 verbunden, weist das Rückschlagventil 23a auf |
| 33 | Atemgasleitung für die Ausatmung, mit dem Y-Stück 22 verbunden, weist das Rückschlagventil 23b auf |

(fortgesetzt)

| | |
|---|---|
| 34 | Abzweigpunkt des Entnahmeschlauchs 52 |
| 35 | Steuergerät, steuert die Fördereinheit 24a und den Narkosemittelverdampfer 31 an, empfängt Signale über die jeweilige Gaskonzentration und Nachrichten von der Signalverarbeitungseinheit 30, generiert bei Bedarf einen Alarm über eine aufgetretene Leckage L |
| 36 | Drucksensor im Beatmungskreislauf 40, misst bevorzugt den am Patienten 20 anliegenden Druck $P_{aw}$ |
| 40 | Beatmungskreislauf, durch den der Patient 20 künstlich beatmet und sediert wird, verbindet den Patienten 20 mit dem Beatmungsgerät 100, umfasst die patientenseitige Koppeleinheit 21, das Y-Stück 22, die Einatmungs-Leitung 32 und die Ausatmungs-Leitung 33 |
| 50 | Gassensor-Anordnung, welche die Konzentration von O2, CO2, N2O und Narkosemittel misst, umfasst die Gassensoren 53 und 54, die Pumpe 55 sowie den Drucksensor 57 |
| 51 | Wasserfalle flussaufwärts von der Gassensor-Anordnung 50, umfasst mindestens eine Membrane, bevorzugt aus PTFE, und einen Tank |
| 52 | Entnahmeschlauch, durch den hindurch eine Atemgasprobe aus dem Beatmungskreislauf 40 entnommen wird, beginnt in einem Abzweigpunkt 34 zwischen der patientenseitigen Koppeleinheit 21 und dem Y-Stück 22 und führt zu der Wasserfalle 51 |
| 53 | Sensor für die O2-Konzentration in der Atemgasprobe |
| 54 | Sensor für die Konzentration von CO2, H2O und Narkosemittel in der Atemgasprobe |
| 55 | Pumpe, welche eine Atemgasprobe in den Entnahmeschlauch 52 hineinsaugt |
| 56 | Abführschlauch, durch den hindurch eine Atemgasprobe wieder in den Beatmungskreislauf 40 eingespeist wird, führt zu einem Einspeisepunkt flussaufwärts von dem Kohlendioxidabsorber 25a |
| 57 | Drucksensor der Gassensor-Anordnung 50, misst den Druck $P_{Cell}$ |
| 58 | Sensor für den Umgebungsdruck $P_{amb}$ |
| 100 | Beatmungsgerät, als Anästhesiegerät ausgestaltet, umfasst den optionalen Narkosemittelverdampfer 31, die Fördereinheit 24a, den Kohlendioxidabsorber 25a, den Drucksensor 36, die Rückschlagventile 23a und 23b, das PEEP-Ventil 24b und den Handbeatmungsbeutel 26 |
| 110 | Mess-System, umfasst Gassensor-Anordnung 50, die Wasserfalle 51 und die Signalverarbeitungseinheit 30 |
| Cov [CO2,O2] | Covarianz zwischen den zeitlichen Verläufen der CO2-Konzentration und der O2-Konzentration |
| DiPhaC | "Differential in Phase Covariance", erstes Leckage-Kriterium, basiert auf vorzeichengleichen Zeitspannen |
| DPC | "Delta Pressure Covariance", zweites Leckage-Kriterium, basiert auf den Veränderungen von Covarianz und Druck |
| L | beispielhafte Leckage im Übergang zwischen dem Entnahmeschlauch 52 und der Wasserfalle 51 |
| $P_{aw}$ | am Patienten 20 anliegender Atmungs- und Beatmungsdruck, vom Sensor 36 gemessen |
| $P_{amb}$ | Umgebungsdruck, vom Sensor 58 gemessen |
| $P_{Cell}$ | Druck am Eingang der Gassensor-Anordnung 50, bevorzugt der Druck relativ zum Umgebungsdruck $P_{amb}$ |
| $P_{cell,abs}$ | absoluter Druck am Eingang der Gassensor-Anordnung 50, vom Sensor 57 gemessen |
| $P_{limit}$ | untere Schranke für die Überprüfung, ob tatsächlich eine Leckage aufgetreten ist |
| T_L, T_L.1, T_L.2 | Zeitraum, in dem zwischen dem Abzweigpunkt 34 und der Gassensor-Anordnung 50 eine Leckage L aufgetreten ist |

**Patentansprüche**

1. Verfahren zur Überwachung eines Mess-Systems (110) für die künstliche Beatmung eines Patienten (20),

   wobei das Mess-System (110)

   - eine Gassensor-Anordnung (50) und
   - eine Gassensor-Fluidführungseinheit (52), insbesondere einen Schlauch, umfasst,

   wobei das Verfahren durchgeführt wird, während mithilfe einer Patienten-Fluidführungseinheit (40) eine Fluidverbindung zwischen

   - dem Patienten (20) und
   - einem medizinischen Gerät (100), insbesondere einem Beatmungsgerät oder einem Anästhesiegerät oder einem Patienten-Monitor,

   hergestellt ist, und
   wobei das Verfahren die automatisch durchgeführten Schritte umfasst, dass eine Gasprobe

   - aus der Patienten-Fluidführungseinheit (40) abgesaugt und
   - durch die Gassensor-Fluidführungseinheit (52) zu der Gassensor-Anordnung (50) geleitet wird,

   unter Verwendung von Messwerten der Gassensor-Anordnung (50)

   - ein Maß für den zeitlichen Verlauf ($CO2_{sk}$) der Konzentration von Kohlendioxid ($CO2$) in der abgesaugten Gasprobe und
   - ein Maß für den zeitlichen Verlauf ($O2_{sk}$) der Konzentration eines von Kohlendioxid ($CO2$) verschiedenen weiteren Gases, welches bevorzugt Sauerstoff ($O2$) umfasst, in der abgesaugten Gasprobe

   berechnet werden,
   unter Verwendung der beiden zeitlichen Konzentrations-Verläufe ($CO2_{sk}$, $O2_{sk}$)

   - ein Maß ($\Delta CO2_{sk}$) für einen zeitlichen Verlauf der zeitlichen Veränderung der CO2-Konzentration und
   - ein Maß ($\Delta O2_{sk}$) für einen zeitlichen Verlauf der zeitlichen Veränderung der Konzentration des weiteren Gases ($O2$)

   berechnet wird,
   nach jeder vorzeichengleichen Zeitspanne,
   das ist eine Zeitspanne, in der die beiden Konzentrations-Veränderungs-Verläufe ($\Delta CO2_{sk}$, $\Delta O2_{sk}$) durchgehend dasselbe Vorzeichen aufweisen, also beide größer null oder beide kleiner null sind,
   gesucht wird und
   dann, wenn mindestens eine solche vorzeichengleiche Zeitspanne detektiert ist,

   - geprüft wird, ob in der oder mindestens einer, bevorzugt in jeder detektierten vorzeichengleichen Zeitspanne die beiden Konzentrations-Veränderungs-Verläufe ($\Delta CO2_{sk}$, $\Delta O2_{sk}$) ein vorgegebenes erstes Le-ckage-Kriterium (DiPhaC) erfüllen, und
   - dann, wenn das erste Leckage-Kriterium (DiPhaC) erfüllt ist, entschieden wird, dass eine Leckage (L) zwischen der Patienten-Fluidführungseinheit (40) und der Gassensor-Anordnung (50) oder ein Indiz für eine solche Leckage (L) aufgetreten ist.

2. Verfahren nach Anspruch 1,
   **dadurch gekennzeichnet, dass**
   das erste Leckage-Kriterium (DiPhaC) von mindestens einem der folgenden Parameter abhängt: von

   - der Länge einer vorzeichengleichen Zeitspanne,
   - der gesamten Länge aller seit einem vorgegebenen Referenz-Zeitpunkt detektierten vorzeichengleichen Zeitspannen,
   - dem jeweiligen arithmetischen Produkt (Prod) der beiden Werte, den die beiden Konzentrations-Veränderungs-

Verläufe ($\Delta CO2_{sk}$, $\Delta O2_{sk}$) an mindestens einem Abtast-Zeitpunkt in der oder einer vorzeichengleichen Zeitspanne annehmen,

- der Summe über mehrere solche arithmetischen Produkte (Prod) von vorzeichengleichen Werten über eine vorzeichengleiche Zeitspanne hinweg,

- der Summe über alle solchen arithmetischen Produkte (Prod) für alle Abtast-Zeitpunkte innerhalb mindestens einer detektierten vorzeichengleichen Zeitspanne, bevorzugt innerhalb aller detektierten vorzeichengleichen Zeitspannen.

3. Verfahren nach einem der vorhergehenden Ansprüche,

   **dadurch gekennzeichnet, dass**
   eine rechnerische Phasenverschiebung des einen Konzentrations-Veränderungs-Verlaufs ($\Delta CO2_{sk}$) relativ zum anderen Konzentrations-Veränderungs-Verlauf ($\Delta O2_{sk}$) dergestalt durchgeführt wird,
   dass nach der Phasenverschiebung das Maximum des CO2-Konzentrations-Veränderungs-Verlaufs ($\Delta CO2_{sk}$) auf denselben Zeitpunkt fällt wie ein Extremum, d.h. Maximum oder Minimum, des anderen Konzentrations-Veränderungs-Verlaufs ($\Delta O2_{sk}$).

4. Verfahren nach einem der vorhergehenden Ansprüche,

   **dadurch gekennzeichnet, dass**
   jede Exspirations-Zeitspanne detektiert wird, also jede Zeitspanne, in welcher der Patient (20) Luft ausatmet, und die Suche nach vorzeichengleichen Zeitspannen nur in den detektierten Exspirations-Zeitspannen durchgeführt wird.

5. Verfahren nach einem der vorhergehenden Ansprüche,

   **dadurch gekennzeichnet, dass**
   das Verfahren die zusätzlichen und ebenfalls automatisch durchgeführten Schritte umfasst, dass
   ein Maß für den zeitlichen Verlauf des Drucks ($P_{cell}$) an einem Messpunkt (57) in oder an der Gassensor-Anordnung (50) oder der Gassensor-Fluidführungseinheit (52) gemessen wird und
   ein Maß für die zeitliche Veränderung des gemessenen Drucks ($P_{cell}$) berechnet wird,
   ein Maß für die Phasenverschiebung zwischen den beiden Konzentrations-Verläufen berechnet wird, insbesondere eine Covarianz (Cov[CO2,O2]),
   ein Maß für die zeitliche Veränderung dieses Phasenverschiebungs-Maßes (Cov[CO2,O2]) berechnet wird,
   geprüft wird, ob ein vorgegebenes zweites Leckage-Kriterium (DPC) erfüllt ist,
   wobei das zweite Leckage-Kriterium (DPC)

   - von der zeitlichen Veränderung des Phasenverschiebungs-Maßes (Cov[CO2,O2]) und
   - von der zeitlichen Veränderung des Drucks ($P_{cell}$) abhängt, und

   dann, wenn detektiert worden ist, dass mindestens eines der beiden Leckage-Kriterien (DiPhaC, DPC) erfüllt ist, entschieden wird, dass eine Leckage (L) zwischen der Patienten-Fluidführungseinheit (40) und der Gassensor-Anordnung (50) oder ein Indiz für eine solche Leckage (L) aufgetreten ist.

6. Verfahren nach Anspruch 5,

   **dadurch gekennzeichnet, dass**
   das zweite Leckage-Kriterium (DPC)

   - von der zeitlichen Veränderung des Phasenverschiebungs-Maßes (Cov[CO2,O2]) an einem ersten Abtast-Zeitpunkt und
   - von der zeitlichen Veränderung des Drucks an einem zweiten Abtast-Zeitpunkt
   abhängt,

   wobei bevorzugt der zeitliche Abstand zwischen den beiden Abtast-Zeitpunkten vom Abstand zwischen

   - dem Messpunkt, an dem der Druck ($P_{cell}$) gemessen wird, und
   - einem Messpunkt, an dem die beiden Konzentrationen (CO2, O2) gemessen werden,

abhängt.

7. Verfahren nach Anspruch 6,
**dadurch gekennzeichnet, dass**
das zweite Leckage-Kriterium (DPC) dann erfüllt ist, wenn

- die zeitliche Veränderung des Phasenverschiebungs-Maßes (Cov[CO2,O2]) an dem ersten Abtast-Zeitpunkt oberhalb einer ersten vorgegebenen Schranke liegt und
- die zeitliche Veränderung des Drucks ($P_{cell}$) an dem zweiten Abtast-Zeitpunkt oberhalb einer zweiten vorgegebenen Schranke liegt.

8. Verfahren nach einem der vorhergehenden Ansprüche,

**dadurch gekennzeichnet, dass**
dann, wenn detektiert ist, dass das erste Leckage-Kriterium (DiPhaC) und / oder das zweite Leckage-Kriterium (DPC) erfüllt ist, automatisch eine Überprüfung durchgeführt wird,
wobei die Überprüfung die Schritte umfasst, dass

- der Schritt, eine Gasprobe aus der Patienten-Fluidführungseinheit (40) abzusaugen, unterbrochen wird,
- mindestens einmal, bevorzugt mehrmals, der Druck ($P_{aw}$) in der Patienten-Fluidführungseinheit (40) und der Druck ($P_{cell}$) an einem Messpunkt (57) in oder an dem Mess-System (110) gemessen werden, während das Absaugen der Gasprobe unterbrochen ist,
- die beiden Drücke ($P_{cell}$, $P_{aw}$) miteinander verglichen werden und
- dann, wenn der Vergleich ein vorgegebenes drittes Leckage-Kriterium ($P_{limit}$) erfüllt, entschieden wird, dass eine Leckage (L) aufgetreten ist.

9. Mess-System (110) für die künstliche Beatmung eines Patienten (20),

wobei das Mess-System (110)

- eine Gassensor-Anordnung (50),
- eine Gassensor-Fluidführungseinheit (52), insbesondere einen Schlauch, und
- eine Signalverarbeitungseinheit (30)
umfasst,

wobei die Gassensor-Fluidführungseinheit (52) mit einer Patienten-Fluidführungseinheit (40) verbunden oder verbindbar ist,
wobei mithilfe der Patienten-Fluidführungseinheit (40) eine Fluidverbindung zwischen

- dem Patienten (20) und
- einem medizinischen Gerät (100), insbesondere einem Beatmungsgerät oder einem Anästhesiegerät oder einem Patienten-Monitor,

hergestellt oder herstellbar ist,
wobei das Mess-System (110) dazu ausgestaltet ist, Gas von der Patienten-Fluidführungseinheit (40) durch die Gassensor-Fluidführungseinheit (52) hindurch zu der Gassensor-Anordnung (50) zu fördern,
wobei die Signalverarbeitungseinheit (30) dazu ausgestaltet ist, automatisch
unter Verwendung von Messwerten der Gassensor-Anordnung (50)

- ein Maß für den zeitlichen Verlauf ($CO2_{sk}$) der Konzentration von Kohlendioxid (CO2) in der abgesaugten Gasprobe und
- ein Maß für den zeitlichen Verlauf ($O2_{sk}$) der Konzentration eines von Kohlendioxid (CO2) verschiedenen weiteren Gases, welches bevorzugt Sauerstoff (O2) umfasst, in der abgesaugten Gasprobe
zu berechnen,

unter Verwendung der beiden zeitlichen Konzentrations-Verläufe ($CO2_{sk}$, $O2_{sk}$)

- ein Maß ($\Delta CO2_{sk}$) für einen zeitlichen Verlauf der zeitlichen Veränderung der CO2-Konzentration und

- ein Maß ($\Delta O2_{sk}$) für einen zeitlichen Verlauf der zeitlichen Veränderung der Konzentration des weiteren Gases (O2)

zu berechnen,

nach jeder vorzeichengleichen Zeitspanne,

das ist eine Zeitspanne, in der die beiden Konzentrations-Veränderungs-Verläufe ($\Delta CO2_{sk}$, $\Delta O2_{sk}$) durchgehend dasselbe Vorzeichen aufweisen, also beide größer null oder beide kleiner null sind,

zu suchen und

dann, wenn mindestens eine solche vorzeichengleiche Zeitspanne detektiert ist,

- zu prüfen, ob in der oder mindestens einer, bevorzugt in jeder detektierten vorzeichengleichen Zeitspanne die beiden Konzentrations-Veränderungs-Verläufe ($\Delta CO2_{sk}$, $\Delta O2_{sk}$) ein vorgegebenes erstes Leckage-Kriterium (DiPhaC) erfüllen, und
- dann, wenn das erste Leckage-Kriterium (DiPhaC) erfüllt ist, zu entscheiden, dass eine Leckage (L) zwischen der Patienten-Fluidführungseinheit (40) und der Gassensor-Anordnung (50) oder ein Indiz für eine solche Leckage (L) aufgetreten ist.

10. Verbindungs-Anordnung für die künstliche Beatmung eines Patienten (20),

    wobei die Verbindungs-Anordnung

    - eine Patienten-Fluidführungseinheit (40) und
    - ein Mess-System (110) nach Anspruch 9
    umfasst,

    wobei mithilfe der Patienten-Fluidführungseinheit (40) eine Fluidverbindung zwischen

    - dem Patienten (20) und
    - einem medizinischen Gerät (100), insbesondere einem Beatmungsgerät oder einem Anästhesiegerät oder einem Patienten-Monitor,

    hergestellt oder herstellbar ist und

    wobei die Gassensor-Fluidführungseinheit (52) wenigstens zeitweise mit der Patienten-Fluidführungseinheit (40) verbunden ist.

11. Medizinisches System, umfassend

    - ein medizinisches Gerät (100), insbesondere ein Beatmungsgerät oder ein Anästhesiegerät oder einen Patienten-Monitor, und
    - eine Verbindungs-Anordnung gemäß Anspruch 10,

    wobei die Verbindungs-Anordnung wenigstens zeitweise eine Fluidverbindung zwischen dem medizinischen Gerät (100) und einem Patienten (20) herstellt und

    wobei bevorzugt das medizinische Gerät (100) dazu ausgestaltet ist,

    - gemessene zeitliche Verläufe ($CO2_{sk}$, $O2_{sk}$) der CO2-Konzentration und der Konzentration des weiteren Gases (O2) von dem Mess-System (110) der Verbindungs-Anordnung zu empfangen und
    - die empfangenen zeitlichen Verläufe ($CO2_{sk}$, $O2_{sk}$) automatisch zu verarbeiten und / oder in einer von einem Menschen wahrnehmbaren Form auszugeben.

**Claims**

1. Method for monitoring a measuring system (110) for the artificial ventilation of a patient (20),

   wherein the measuring system (110) comprises

   - a gas sensor arrangement (50) and

- a gas sensor fluid-guiding unit (52), in particular a hose,

wherein the method is performed while, with the aid of a patient-side fluid-guiding unit (40), a fluidic connection between

- the patient (20) and
- a medical appliance (100), in particular a ventilator or an anaesthesia appliance or a patient monitor, is established, and

wherein the method comprises the automatically performed steps of a gas sample

- being suctioned from the patient-side fluid-guiding unit (40) and
- being guided through the gas sensor fluid-guiding unit (52) to the gas sensor arrangement (50),

using measured values of the gas sensor arrangement (50) to calculate

- a measure of the time curve (CO2sk) of the concentration of carbon dioxide (CO2) in the suctioned gas sample and
- a measure of the time curve (O2sk) of the concentration of a further gas, different from carbon dioxide (CO2), which preferably comprises oxygen (O2), in the suctioned gas sample,

using the two time-related concentration curves (CO2sk, O2sk) to calculate

- a measure ($\Delta$CO2sk) of a time curve of the temporal change of the CO2 concentration and
- a measure ($\Delta$O2sk) of a time curve of the temporal change of the concentration of the further gas (O2),

searching for each same-sign time period,
that is a time period in which the two concentration change curves ($\Delta$CO2sk, $\Delta$O2sk) have the same sign throughout, i.e. both are greater than zero or both are less than zero,
and, if at least one such same-sign time period is detected,

- checking whether the two concentration change curves ($\Delta$CO2sk, $\Delta$O2sk) meet a predefined first leakage criterion (DiPhaC) in the or at least one, preferably in each, detected same-sign time period, and,
- if the first leakage criterion (DiPhaC) is met, concluding that a leakage (L) has occurred between the patient-side fluid-guiding unit (40) and the gas sensor arrangement (50) or that an indication of such a leakage (L) has occurred.

2. Method according to Claim 1,

    **characterized in that**
    the first leakage criterion (DiPhaC) depends on at least one of the following parameters:

    - the duration of a same-sign time period,
    - the total duration of all same-sign time periods detected since a predefined reference time,
    - the respective arithmetic product (Prod) of the two values that the two concentration change curves ($\Delta$CO2sk, $\Delta$O2sk) assume at at least one sampling time in the or a same-sign time period,
    - the sum of a plurality of such arithmetic products (Prod) of same-sign values over the course of a same-sign time period,
    - the sum of all such arithmetic products (Prod) for all sampling times within at least one detected same-sign time period, preferably within all the detected same-sign time periods.

3. Method according to one of the preceding claims,

    **characterized in that**
    a calculated phase shift of one concentration change curve ($\Delta$CO2sk) relative to the other concentration change curve ($\Delta$O2sk) is performed in such a way that,
    after the phase shift, the maximum of the CO2 concentration change curve ($\Delta$CO2sk) occurs at the same time as an extreme, i.e. maximum or minimum, of the other concentration change curve ($\Delta$O2sk).

4. Method according to one of the preceding claims,

    **characterized in that**
    each expiration time period, i.e. each time period in which the patient (20) exhales air, is detected, and
    the search for same-sign time periods is performed only in the detected expiration time periods.

5. Method according to one of the preceding claims,

    **characterized in that**
    the method comprises the additional and likewise automatically performed steps in which
    a measure of the time curve of the pressure (Pcell) is measured at a measuring point (57) in or at the gas sensor
    arrangement (50) or the gas sensor fluid-guiding unit (52), and
    a measure of the temporal change of the measured pressure (Pcell) is calculated,
    a measure of the phase shift between the two concentration curves is calculated, in particular a covariance
    (Cov[CO2,O2]),
    a measure of the temporal change of this phase shift measure (Cov[CO2,O2]) is calculated,
    a check is made as to whether a predefined second leakage criterion (DPC) is met,
    wherein the second leakage criterion (DPC) depends

        - on the temporal change of the phase shift measure (Cov[CO2,O2]) and
        - on the temporal change of the pressure (Pcell),

    and, if it has been detected that at least one of the two leakage criteria (DiPhaC), DPC) is met,
    it is concluded that a leakage (L) has occurred between the patient-side fluid-guiding unit (40) and the gas
    sensor arrangement (50) or that an indication of such a leakage (L) has occurred.

6. Method according to Claim 5,

    **characterized in that**
    the second leakage criterion (DPC) depends

        - on the temporal change of the phase shift measure (Cov[CO2,O2]) at a first sampling time, and
        - on the temporal change of the pressure at a second sampling time,

    wherein the time interval between the two sampling times preferably depends on the interval between

        - the measuring point at which the pressure (Pcell) is measured and
        - a measuring point at which the two concentrations (CO2, O2) are measured.

7. Method according to Claim 6,
    **characterized in that**
    the second leakage criterion (DPC) is met when

        - the temporal change of the phase shift measure (Cov[CO2,O2]) at the first sampling time is above a first
        predefined limit and
        - the temporal change of the pressure (Pcell) at the second sampling time is above a second predefined limit.

8. Method according to one of the preceding claims,

    **characterized in that**,
    if it is detected that the first leakage criterion (DiPhaC) and/or the second leakage criterion (DPC) are/is met, a
    check is automatically performed,
    wherein the check comprises the steps of

        - interrupting the step of suctioning a gas sample from the patient-side fluid-guiding unit (40),
        - measuring at least once, preferably several times, the pressure (Paw) in the patient-side fluid-guiding unit
        (40) and the pressure (Pcell) at a measuring point (57) in or at the measuring system (110) while the
        suctioning of the gas sample is interrupted,

- comparing the two pressures (Pcell, Paw) with each other, and,
- if the comparison meets a predefined third leakage criterion (Plimit), concluding that a leakage (L) has occurred.

9. Measuring system (110) for the artificial ventilation of a patient (20),

wherein the measuring system (110) comprises

- a gas sensor arrangement (50),
- a gas sensor fluid-guiding unit (52), in particular a hose, and
- a signal processing unit (30),

wherein the gas sensor fluid-guiding unit (52) is connected or connectable to a patient-side fluid-guiding unit (40), wherein, with the aid of the patient-side fluid-guiding unit (40), a fluidic connection is established or can be established between

- the patient (20) and
- a medical appliance (100), in particular a ventilator or an anaesthesia appliance or a patient monitor,

wherein the measuring system (110) is configured to deliver gas from the patient-side fluid-guiding unit (40) through the gas sensor fluid-guiding unit (52) to the gas sensor arrangement (50),
wherein the signal processing unit (30) is configured to automatically calculate,
using measured values of the gas sensor arrangement (50),

- a measure of the time curve (CO2sk) of the concentration of carbon dioxide (CO2) in the suctioned gas sample, and
- a measure of the time curve (O2sk) of the concentration of a further gas, different from carbon dioxide (CO2), which preferably comprises oxygen (O2), in the suctioned gas sample, and,

using the two time-related concentration curves (CO2sk, O2sk),

- a measure ($\Delta$CO2sk) of a time curve of the temporal change of the CO2 concentration and
- a measure ($\Delta$O2sk) of a time curve of the temporal change of the concentration of the further gas (O2),

to search for each same-sign time period,
that is a time period in which the two concentration change curves ($\Delta$CO2sk, $\Delta$O2sk) have the same sign throughout, i.e. both are greater than zero or both are less than zero,
and, if at least one such same-sign time period is detected,

- to check whether the two concentration change curves ($\Delta$CO2sk, $\Delta$O2sk) meet a predefined first leakage criterion (DiPhaC) in the or at least one, preferably in each, detected same-sign time period, and,
- if the first leakage criterion (DiPhaC) is met, to conclude that a leakage (L) has occurred between the patient-side fluid-guiding unit (40) and the gas sensor arrangement (50) or that an indication of such a leakage (L) has occurred.

10. Connection arrangement for the artificial ventilation of a patient (20),

wherein the connection arrangement comprises

- a patient-side fluid-guiding unit (40) and
- a measuring system (110) according to Claim 9,

wherein, with the aid of the patient-side fluid-guiding unit (40), a fluidic connection is established or can be established between

- the patient (20) and
- a medical appliance (100), in particular a ventilator or an anaesthesia appliance or a patient monitor, and

wherein the gas sensor fluid-guiding unit (52) is at least temporarily connected to the patient-side fluid-guiding unit (40).

11. Medical system comprising

- a medical appliance (100), in particular a ventilator or an anaesthesia appliance or a patient monitor, and
- a connection arrangement according to Claim 10,

wherein the connection arrangement at least temporarily establishes a fluidic connection between the medical appliance (100) and a patient (20), and
wherein the medical appliance (100) is preferably configured

- to receive measured time curves (CO2sk, O2sk) of the CO2 concentration and of the concentration of the further gas (O2) from the measuring system (110) of the connection arrangement and
- to automatically process the received time curves (CO2sk, O2sk) and/or to output them in a form perceptible to a human.

**Revendications**

1. Procédé de surveillance d'un système de mesure (110) ciblant la respiration artificielle d'un patient (20),

ledit système de mesure (110) comprenant

- un agencement (50) détecteur de gaz et
- une unité (52), en particulier un tuyau souple de guidage de fluide vers ledit détecteur de gaz,

sachant que le procédé est mis en oeuvre alors même qu'à l'aide d'une unité (40) de guidage de fluide vers le patient, une liaison fluidique est instaurée entre

- le patient (20) et
- un appareil médical (100), notamment un respirateur ou un appareil d'anesthésie, voire un moniteur dudit patient, et

sachant que ledit procédé inclut les étapes exécutées automatiquement, consistant
en ce qu'un échantillon gazeux

- est aspiré à partir de l'unité (40) de guidage de fluide vers le patient et
- est dirigé vers l'agencement (50) détecteur de gaz par l'intermédiaire de l'unité (52) de guidage de fluide vers ledit détecteur de gaz,

en ce qu'en utilisant des valeurs de mesure dudit agencement (50) détecteur de gaz, il s'opère un calcul

- d'un paramètre estimatif affecté au profil temporel ($CO_{2sk}$) de la concentration de dioxyde de carbone ($CO_2$) dans l'échantillon gazeux aspiré et
- d'un paramètre estimatif affecté au profil temporel ($O_{2sk}$) de la concentration, dans ledit échantillon gazeux aspiré, d'un gaz additionnel différant du dioxyde de carbone ($CO_2$) et renfermant préférentiellement de l'oxygène ($O_2$),

en ce qu'en utilisant les deux profils temporels de concentration ($CO_{2sk}$, $O_{2sk}$), il s'opère un calcul

- d'un paramètre estimatif ($\Delta CO_{2sk}$) affecté à un profil temporel de la variation de la concentration de CO2 dans le temps et
- d'un paramètre estimatif ($\Delta O_{2sk}$) affecté à un profil temporel de la variation de la concentration du gaz additionnel ($O_2$) dans le temps,

en ce qu'il s'opère une recherche de chaque période de signe identique,
à savoir une période durant laquelle les deux profils ($\Delta CO_{2sk}$, $\Delta O_{2sk}$) de variation de concentration présentent constamment le même signe, c'est-à-dire sont l'un et l'autre supérieurs à zéro, ou l'un et l'autre inférieurs à zéro, et en ce que, lorsqu'au moins une telle période de signe identique est détectée,

- il est vérifié si durant la ou durant au moins une, de préférence durant chaque période de signe identique détectée, les deux profils ($\Delta CO_{2sk}$, $\Delta O_{2sk}$) de variation de concentration satisfont à un premier critère de fuite (DiPhaC) préétabli et
- ensuite, lorsqu'il est satisfait audit premier critère de fuite (DiPhaC), il est décidé qu'une fuite (L), ou un indice d'une telle fuite (L), est survenu(e) entre l'unité (40) de guidage de fluide vers le patient et l'agencement (50) détecteur de gaz.

2. Procédé selon la revendication 1,

**caractérisé par le fait que**
le premier critère de fuite (DiPhaC) dépend d'au moins l'un des paramètres suivants :

- de la durée d'une période de signe identique,
- de la durée cumulée de toutes les périodes de signe identique détectées depuis un point temporel de référence préétabli,
- du produit arithmétique (Prod) respectif des deux valeurs que les deux profils ($\Delta CO_{2sk}$, $\Delta O_{2sk}$) de variation de concentration prennent, en au moins un point temporel de balayage exploratoire, durant la ou durant une période de signe identique,
- de la somme se rapportant à plusieurs produits arithmétiques (Prod) de valeurs de même signe de ce type, couvrant une période de signe identique,
- de la somme se rapportant à tous les produits arithmétiques de ce type, concernant tous les points temporels de balayage exploratoire dans les limites d'au moins une période de signe identique détectée, de préférence dans les limites de toutes les périodes de signe identique détectées.

3. Procédé selon l'une des revendications précédentes,

**caractérisé par le fait**
**qu'**un décalage de phase de l'un ($\Delta CO_{2sk}$) des profils de variation de concentration par rapport à l'autre profil ($\Delta O_{2sk}$) de variation de concentration, obtenu par calcul, est effectué de façon telle
**qu'**à l'issue dudit décalage de phase, le maximum du profil ($\Delta CO_{2sk}$) de variation de concentration de $CO_2$ se présente au même point temporel qu'un extremum, c'est-à-dire un maximum ou un minimum de l'autre profil ($\Delta O_{2sk}$) de variation de concentration.

4. Procédé selon l'une des revendications précédentes,

**caractérisé par le fait**
**qu'**il s'opère une détection de chaque période expiratoire, c'est-à-dire de chaque période durant laquelle le patient (20) expire de l'air, et
**que** la recherche de périodes de signe identique est effectuée uniquement durant les périodes expiratoires détectées.

5. Procédé selon l'une des revendications précédentes,

**caractérisé par le fait que**
ledit procédé inclut les étapes supplémentaires, pareillement exécutées en mode automatique, consistant
en ce qu'un paramètre estimatif affecté au profil temporel de la pression ($P_{cell}$) est mesuré, en un point de mesure (57), dans ou sur l'agencement (50) détecteur de gaz ou l'unité (52) de guidage de fluide vers ledit détecteur de gaz et
il s'opère un calcul d'un paramètre estimatif affecté à la variation temporelle de la pression ($P_{cell}$) mesurée,
en ce qu'il s'opère un calcul d'un paramètre estimatif affecté au décalage de phase entre les deux profils de concentration, en particulier une covariance ($Cov[CO_2, O_2]$),
en ce qu'il s'opère un calcul d'un paramètre estimatif affecté à la variation temporelle de ce paramètre estimatif ($Cov[CO_2, O_2]$) dudit décalage de phase, en ce qu'il est vérifié s'il est satisfait à un deuxième critère de fuite (DPC) préétabli,
lequel deuxième critère de fuite (DPC) dépend

- de la variation temporelle du paramètre estimatif (Cov[$CO_2$, $O_2$]) du décalage de phase et
- de la variation temporelle de la pression ($P_{cell}$), et

ensuite, lorsqu'il a été détecté qu'il est satisfait à au moins l'un des deux critères de fuite (DiPhaC, DPC), il est décidé qu'une fuite (L), ou un indice d'une telle fuite (L), est survenu(e) entre l'unité (40) de guidage de fluide vers le patient et l'agencement (50) détecteur de gaz.

**6.** Procédé selon la revendication 5,

**caractérisé par le fait que**
le deuxième critère de fuite (DPC) dépend

- de la variation temporelle du paramètre estimatif (Cov[$CO_2$, $O_2$]) du décalage de phase, en un premier point temporel de balayage exploratoire, et
- de la variation temporelle de la pression en un second point temporel de balayage exploratoire,

sachant, de préférence, que l'intervalle de temps entre les deux points temporels de balayage exploratoire dépend de l'espacement entre

- le point de mesure auquel la pression ($P_{cell}$) est mesurée et
- un point de mesure auquel les deux concentrations ($CO_2$, $O_2$) sont mesurées.

**7.** Procédé selon la revendication 6,
**caractérisé par le fait**
**qu'**il est satisfait au deuxième critère de fuite (DPC) lorsque

- la variation temporelle du paramètre estimatif (Cov[$CO_2$, $O_2$]) du décalage de phase se situe au-dessus d'une première limite préétablie, au premier point temporel de balayage exploratoire, et
- la variation temporelle de la pression ($P_{cell}$) se situe au-dessus d'une seconde limite préétablie au second point temporel de balayage exploratoire.

**8.** Procédé selon l'une des revendications précédentes,
**caractérisé par le fait**
**qu'**un contrôle est effectué automatiquement lorsqu'il est détecté qu'il est satisfait au premier critère de fuite (DiPhaC) et/ou au deuxième critère de fuite (DPC), lequel contrôle inclut les étapes consistant

- à interrompre l'étape d'aspiration d'un échantillon gazeux à partir de l'unité (40) de guidage de fluide vers le patient,
- à mesurer au moins une fois, de préférence plusieurs fois au cours de l'interruption de l'aspiration dudit échantillon gazeux, dans ou sur le système de mesure (110), la pression ($P_{aw}$) régnant dans ladite unité (40) de guidage de fluide vers le patient et la pression ($P_{cell}$) régnant en un point de mesure (57),
- à comparer l'une à l'autre les deux pressions ($P_{cell}$, $P_{aw}$) et
- à décider qu'une fuite (L) est survenue lorsque la comparaison satisfait à un troisième critère de fuite ($P_{limit}$) préétabli.

**9.** Système de mesure (110) ciblant la respiration artificielle d'un patient (20),

lequel système de mesure (110) comprend

- un agencement (50) détecteur de gaz,
- une unité (52), en particulier un tuyau souple de guidage de fluide vers ledit détecteur de gaz et
- une unité (30) de traitement de signaux,

ladite unité (52) de guidage de fluide vers ledit détecteur de gaz étant, ou pouvant être reliée à une unité (40) de guidage de fluide vers le patient,
sachant qu'à l'aide de ladite unité (40) de guidage de fluide vers le patient, une liaison fluidique est, ou peut être instaurée entre

- le patient (20) et
- un appareil médical (100), notamment un respirateur ou un appareil d'anesthésie, voire un moniteur dudit patient,

ledit système de mesure (110) étant conçu pour acheminer du gaz vers l'agencement (50) détecteur de gaz à partir de l'unité (40) de guidage de fluide vers le patient, en parcourant intégralement l'unité (52) de guidage de fluide vers ledit détecteur de gaz,

sachant que l'unité (30) de traitement de signaux est conçue pour calculer automatiquement, en utilisant des valeurs de mesure dudit agencement (50) détecteur de gaz,

- un paramètre estimatif affecté au profil temporel ($CO_{2sk}$) de la concentration de dioxyde de carbone ($CO_2$) dans l'échantillon gazeux aspiré et
- un paramètre estimatif affecté au profil temporel ($O_{2sk}$) de la concentration, dans ledit échantillon gazeux aspiré, d'un gaz additionnel, différant du dioxyde de carbone ($CO_2$) et renfermant préférentiellement de l'oxygène ($O_2$),

pour calculer, en utilisant les deux profils temporels de concentration ($CO_{2sk}$, $O_{2sk}$),

- un paramètre estimatif ($\Delta CO_{2sk}$) affecté à un profil temporel de la variation de la concentration de $CO_2$ dans le temps et
- un paramètre estimatif ($\Delta O_{2sk}$) affecté à un profil temporel de la variation de la concentration de gaz additionnel ($O_2$) dans le temps,

pour rechercher chaque période de signe identique,
à savoir une période durant laquelle les deux profils ($\Delta CO_{2sk}$, $\Delta O_{2sk}$) de variation de concentration présentent constamment le même signe, c'est-à-dire sont l'un et l'autre supérieurs à zéro, ou l'un et l'autre inférieurs à zéro, et pour vérifier ensuite, lorsqu'au moins un telle période de signe identique est détectée,

- si durant la ou durant au moins une, de préférence durant chaque période de signe identique détectée, les deux profils ($\Delta CO_{2sk}$, $\Delta O_{2sk}$) de variation de concentration satisfont à un premier critère de fuite (DiPhaC) préétabli et
- pour décider à un stade successif, lorsqu'il est satisfait audit premier critère de fuite (DiPhaC), qu'une fuite (L) ou un indice d'une telle fuite (L) est survenu(e) entre l'unité (40) de guidage de fluide vers le patient et l'agencement (50) détecteur de gaz.

**10.** Dispositif de liaison ciblant la respiration artificielle d'un patient (20),

ledit dispositif de liaison comprenant

- une unité (40) de guidage de fluide vers le patient et
- un système de mesure (110) conforme à la revendication 9,

sachant qu'à l'aide de ladite unité (40) de guidage de fluide vers le patient, une liaison fluidique est, ou peut être instaurée entre

- le patient (20) et
- un appareil médical (100), notamment un respirateur ou un appareil d'anesthésie, voire un moniteur dudit patient, et

sachant que l'unité (52) de guidage de fluide vers le détecteur de gaz est reliée, au moins périodiquement, à ladite unité (40) de guidage de fluide vers le patient.

**11.** Système médical comprenant

- un appareil médical (100), notamment un respirateur ou un appareil d'anesthésie, voire un moniteur de patient, et
- un dispositif de liaison conforme à la revendication 10,

sachant que ledit dispositif de liaison instaure, au moins périodiquement, une liaison fluidique entre l'appareil médical (100) et un patient (20), et

sachant que, de préférence, ledit appareil médical (100) est conçu

- pour recevoir, en provenance du système de mesure (110) dudit dispositif de liaison, des profils temporels mesurés ($CO_{2sk}$, $O_{2sk}$) de la concentration de $CO_2$ et de la concentration du gaz additionnel ($O_2$), et
- pour traiter automatiquement les profils temporels ($CO_{2sk}$, $O_{2sk}$) reçus, et/ou pour les restituer sous une forme perceptible par un être humain.

FIG. 1

FIG. 2

EP 3 903 863 B1

FIG. 3

EP 3 903 863 B1

FIG. 4

EP 3 903 863 B1

FIG. 5b

FIG. 5a

FIG. 6a            FIG. 6b

EP 3 903 863 B1

FIG. 7

FIG. 8

EP 3 903 863 B1

FIG. 9

DiPhaC

DiPhaC

DiPhaC

$P_{cell}$

$P_{cell}$

$P_{cell}$

Atemphasen-Nr.

FIG. 10

FIG. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102017011625 A1
- US 8033280 B2
- EP 1961439 A1
- WO 2004076944 A2
- DE 102007046533 B3
- DE 102009024040 A1